# EUROPEAN PATENT APPLICATION

(11) **EP 4 113 120 A1**
(43) Date of publication of application: **04.01.2023**
(21) Application number: 21182155.8
(22) Date of filing: 28.06.2021
(51) Int. Cl.: G01N 33/68

(54) **METHOD OF CHARACTERIZING THE BINDING CHARACTERISTICS BETWEEN A PEPTIDE OF INTEREST AND MHC MOLECULES**

(71) Applicant: Immatics Biotechnologies GmbH, 72076 Tübingen (DE)
(72) Inventor: Schräder, Christoph, 72076 Tübingen (DE); Schuster, Heiko, 72076 Tübingen (DE); Freudenmann, Lena, 72076 Tübingen (DE); Goldfinger, Valentina, 72076 Tübingen (DE); Kowalewski, Daniel, 72076 Tübingen (DE); Yousef, Sara, 72076 Tübingen (DE); Manz, Timo, 72076 Tübingen (DE); Mijosek, Vedrana, 72076 Tübingen (DE); Römer, Michael, 72076 Tübingen (DE); Weinschenk, Toni, 72076 Tübingen (DE)
(74) Representative: Michalski Hüttermann & Partner Patentanwälte mbB

(57) **Abstract**

The present invention relates to a method of characterizing the binding characteristics between a peptide of interest and MHC molecules of a given cell type, the method comprising the steps of: (i) Providing two or more cells characterized by displaying, on their surface, MHC molecules, (ii) dispensing the two or more cells in two or more vessels, so that each vessel comprises one or more cells, (iii) adding, to the different vessels, different variants of a peptide of interest, wherein the variants of said peptide are labeled and have the same amino acid sequence, yet differ from one another in the type of labeling and their concentration, and exposing the cells thereto so as to form, in the different vessels, peptide-MHC complexes on the surface of the cells, (iv) isolating the thus formed peptide-MHC complexes and (v) determining the concentration of the different peptide-MHC complexes formed (Fig 1).

## Description

### Field of the invention

The present application relates to a method of characterizing the binding characteristics between a peptide of interest and MHC molecules.

### Incorporation by Reference

All publications, patents, patent applications and other documents cited in this application are hereby incorporated by reference in their entireties for all purposes to the same extent as if each individual publication, patent, patent application or other document were individually indicated to be incorporated by reference for all purposes. In the event that there are any inconsistencies between the teachings of one or more of the references incorporated herein and the present disclosure, the teachings of the present specification are intended.

### Background

The major histocompatibility complex (MHC) is a gene cluster on chromosome 6 which is common to most vertebrates encoding for different genes, which play a fundamental role in histocompatibility and the adaptive immune system. In humans this cluster is often also commonly referred to as human leukocyte antigen (HLA). MHC class I molecules are expressed on all cells of a mammal with the exception of erythrocytes. Their main function is to present short peptides derived from intracellular or endocytosed proteins to cytotoxic T lymphocytes (CTLs) (Boniface and Davis, 1995; Goldberg and Rizzo, 2015b; Gruen and Weissman, 1997; Rock and Shen, 2005). CTLs express CD8 co-receptors, in addition to T cell receptors (TCRs). When a CTL's CD8 receptor docks to an MHC class I molecule on a target cell, if the CTL's TCR fits the epitope represented by the complex of MHC class I molecule and presented peptide, the CTL triggers the target cell lysis by either releasing a cargo of cytolytic enzymes or rendering the cell to undergo programmed cell death by apoptosis (Boniface and Davis, 1995; Delves and Roitt, 2000; Lustgarten et al., 1991). Thus, MHC class I helps mediate cellular immunity, a primary means to address intracellular pathogens, such as viruses and some bacteria, including bacterial L forms or bacterial genera Shigella and Rickettsia (Goldberg and Rizzo, 2015b; Madden et al., 1993; Ray et al., 2009). Furthermore, this process is also of utmost importance for the immunological response and defense against neoplastic diseases such as cancer (Coley, 1991; Coulie et al., 2014; Urban and Schreiber, 1992).

Heterodimeric MHC class I molecules are composed of a polymorphic heavy α-subunit encoded within the MHC gene cluster and a small invariant beta-2-microglobulin (β2m) subunit whose gene is located outside of the MHC locus on chromosome 15. The polymorphic α chain encompasses an N-terminal extracellular region composed by three domains, α1, α2, and α3, a transmembrane helix accomplishing cell surface attachment of the MHC molecule, and a short cytoplasmic tail. Two domains, α1 and α2, form a peptide-binding groove between two long α-helices, whereas the floor of the groove is formed by eight β-strands. The Immunoglobulin-like domain α3 is involved in the interaction with the CD8 co-receptor. The invariant β2m provides stability of the complex and participates in recognition of the peptide-MHC class I complex by CD8 co-receptors. β2m is non-covalently bound to the α-subunit. It is held by several pockets on the floor of the peptide-binding groove. Amino acid (AA) side chains that vary widely between different human HLA alleles fill up the central and widest portion of the binding groove, while conserved side chains are clustered at the narrower ends of the groove. The polymorphic amino acid residues authoritatively define the biochemical properties of peptides which can be bound by the respective HLA molecule (Boniface and Davis, 1995; Falk et al., 1991; Goldberg and Rizzo, 2015a; Rammensee et al., 1995).

In humans, the MHC class I gene cluster is characterized by polymorphism and polygenicity. Each chromosome encodes one HLA-A, -B, and -C allele together constituting the HLA class I haplotype. Consequently, up to six different classical HLA class I molecules can be expressed on the surface of an individual's cells; an exemplary combination of HLA-A, -B, and -C allotypes is given in the table below. In June 2021, the IPD-IMGT/HLA Database (release 3.44.1, 2021-06-11) comprised a total of 6,766 HLA-A alleles (4,064 proteins), 7,967 HLA-B alleles (4,962 proteins), and 6,621 HLA-C alleles (3,831 proteins) (Robinson et al., 2015).

MHC molecules are tissue antigens that allow the immune system to bind to, recognize, and tolerate itself (autorecognition). MHC molecules also function as chaperones for intracellular peptides that are complexed with MHC heterodimers and presented to T cells as potential foreign antigens (Felix and Allen, 2007; Stern and Wiley, 1994).

MHC molecules interact with TCRs and different co-receptors to optimize binding conditions for the TCR-antigen interaction, in terms of antigen binding affinity and specificity, and signal transduction effectiveness (Boniface and Davis, 1995; Gao et al., 2000; Lustgarten et al., 1991). Essentially, the MHC-peptide complex is a complex of auto-antigen/allo-antigen. Upon binding, T cells should in principle tolerate the auto-antigen, but activate when exposed to the allo-antigen. Disease states (especially autoimmunity) occur when this principle is disrupted (Basu et al., 2001; Felix and Allen, 2007; Whitelegg et al., 2005).

On MHC class I, a cell normally presents cytosolic peptides, mostly self-peptides derived from protein turnover and defective ribosomal products (Goldberg and Rizzo, 2015b; Schwanhausser et al., 2011, 2013; Yewdell, 2003; Yewdell et al., 1996). These peptides typically have an extended conformation and oftentimes a length of 8 to 12 amino acids residues, but accommodation of slightly longer versions is feasible as well (Guo et al., 1992; Madden et al., 1993; Rammensee, 1995). During infection with intracellular pathogens including viruses and microorganisms as well as in the course of cancerous transformation, proteins of foreign origin or associated with malignant transformation are also degraded in the proteasome, loaded onto MHC class I molecules, and further displayed on the cell surface (Goldberg and Rizzo, 2015b; Madden et al., 1993; Urban and Schreiber, 1992). Moreover, a phenomenon designated as cross-presentation accomplishes loading of extracellular antigens on MHC class I enabling activation of naive CTLs by dendritic cells (DCs) (Rock and Shen, 2005). T cells can detect a peptide displayed at 0.1%-1% of the MHC molecules and still evoke an immune reaction (Davenport et al., 2018; Sharma and Kranz, 2016; Siller-Farfan and Dushek, 2018; van der Merwe and Dushek, 2011).

Depending on their origin, the peptides displayed by MHC class I are called "tumor-associated peptides" (*TUMAPs*)*,* "virus-derived peptides" or, more general, "pathogen-derived peptides" (Coulie et al., 2014; Freudenmann et al., 2018; Kirner et al., 2014; Urban and Schreiber, 1992).

The interplay between MHC class I, peptides presented thereby, and T cell receptors has been used as a leverage for therapeutic interventions, including (i) vaccination, (ii) TCR therapy, and (iii) adoptive T-cell therapy (Dahan and Reiter, 2012; He et al., 2019; Hilf et al., 2019; Kuhn et al., 2019; Rosenberg et al., 2011; Velcheti and Schalper, 2016).

Vaccination with TUMAPs has been used to prime and activate the immune system against cancer. The underlying activation cascade comprises vaccination, priming, proliferation, and elimination. In the vaccination step, TUMAPs are administered intradermally together with adjuvants/immunomodulators to create an inflammatory milieu and recruit and mature immune cells (dendritic cells). In the priming step, TUMAPs are again administered and bind to dermal DCs, where they are loaded onto MHC class I molecules. The DCs then migrate into the lymph nodes, where they activate ("prime") naive T cells specifically recognizing the TUMAPs used in the vaccine *via* their TCR. Once T cells are primed, their number increases rapidly (clonal proliferation). They leave the lymph nodes and begin searching for tumor cells displaying exactly the same TUMAP on their MHCs by which they were activated in the process of priming. Once a respective target cell is found, the T cell mounts a cytolytic/apoptotic attack against the tumor cells (Hilf et al., 2019; Kirner et al., 2014; Molenkamp et al., 2005) .

In adoptive T-cell therapy, a patient's own T cells are isolated, optionally enriched for clones with desired antigen specificity, expanded *in vitro,* and re-infused into the patient. Isolated autologous T cells can further be modified to express a TCR that has been engineered to recognize a specific pathogen-derived or tumor-associated peptide. In such way, these T cells are taught to bind to cells at the site of disease and exert a cytolytic/apoptotic attack against these target cells. Moreover, it is possible to incorporate co-stimulatory molecules such as CD40 ligand into these T cells equipped with chimeric antigen receptors (CAR) to further enhance the triggered anti-tumor immune response (Kuhn et al., 2019; Rosenberg et al., 2011).

An alternative category of therapeutic approaches employs engineered, soluble TCRs recognizing a specific pathogen-derived or tumor-associated peptide when presented on MHC (Dahan and Reiter, 2012; He et al., 2019). These TCRs may carry an immunomodulatory moiety that is capable of engaging T cells, like an antibody fragment that has affinity to CD3, a molecule that is abundant on T cells. By this mechanism, T cells are redirected to the site of disease and mount a cytolytic/apoptotic attack against the target cells (Chang et al., 2016; Dao et al., 2015; He et al., 2019). A major advantage of soluble TCRs over antibody-based (immuno)therapies is the expansion of the potential target repertoire to intracellular proteins instead of being limited to cell surface antigens accessible to classical antibody formats (Dahan and Reiter, 2012; He et al., 2019).

In order to develop therapeutic entities that recognize peptide-MHC complexes, suitable assays are necessary to characterize the binding properties of such entities to the peptide-MHC complexes, or the cells presenting them. It is also desirable to be able to determine the potency of such entities, i.e., in terms of cell killing activity. It is also desirable to be able to establish dose-response curves to determine dose-dependent effects, such as half maximal inhibitory concentration (IC₅₀). It is also desirable to be able to use a standardized cell line, in order to achieve maximum reproducibility for a given peptide to be investigated, as well between different peptides. Because the total number of peptides that can serve as potential targets is almost unlimited, with the major share of such peptides not yet discovered, it is also desirable to have an assay system that can be used for all conceivable peptides that can be displayed by MHC.

### Brief description of the Figures

Fig. 1 shows a general principle of some elements of the method according to the invention.
Fig. 2 shows the differentiation of selected isotopically KLK3 peptide variants ("isotopologues") from each other either on the level of the precursor ion (full MS) or the resulting fragment ions (MS/MS; exemplary for 2+ precursor ions with 618.30 m/z).
Fig. 3 shows the generation of a calibration curve which has been acquired for selected isotopically labeled variants ("isotopologues") of a given peptide (in this example, derived from KLK3 (SLFHPEDTGQV), n = 6). The different variants have been identified in the mass spectrometry readout.
   Based on such calibration curve, MS signals measured in a given experiment can be converted into concentrations of the respective isotopically labeled variants. The underlying methods are described, *inter alia,* in WO2016107740A1, the content of which is incorporated herein by reference for enablement purposes only.

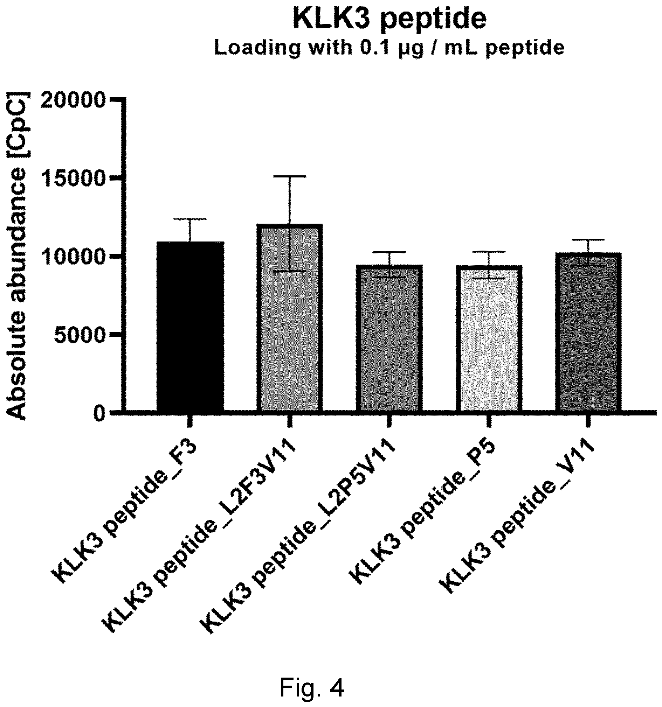

Fig. 4. Pooled samples (n=5) show high reproducibility at a CV of ∼ 11% ("biological" replicates) when loading T2 cells with different isotopologues at the same concentration.

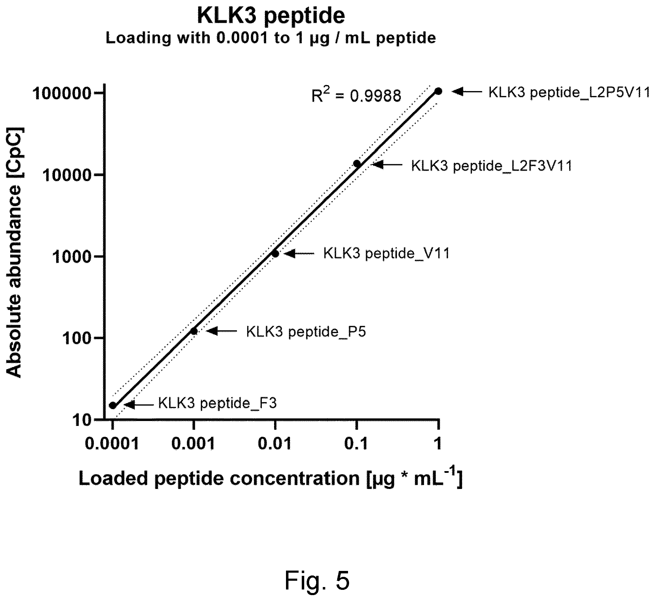

Fig. 5 shows a titration curve between (a) peptide of interest (differently labeled peptide variants in different concentrations) and (b) resulting MS signal obtained for the peptide of interest, normalized to cell count using T2 cells.

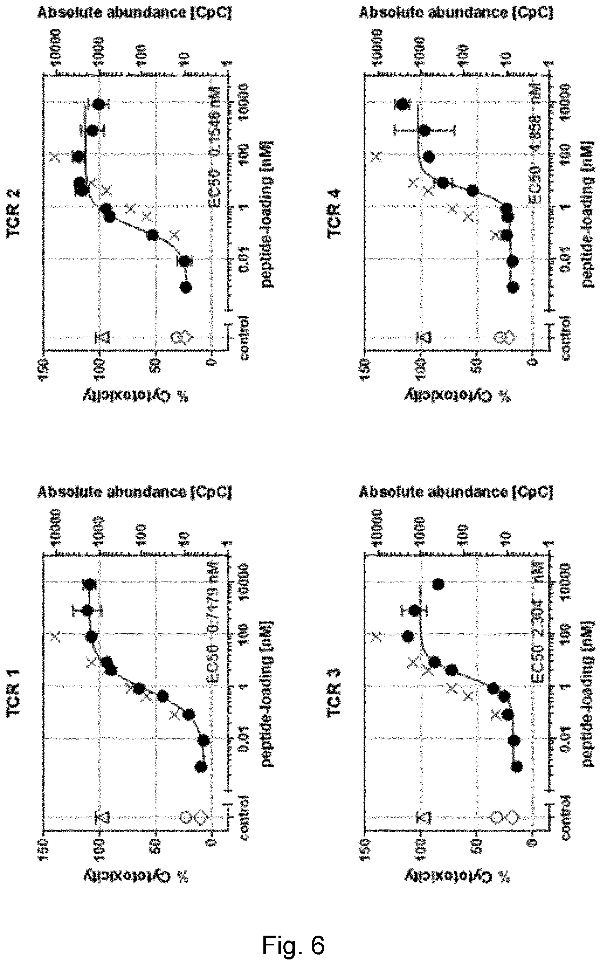

Fig. 6 Cytotoxicity assay. Functional avidity (EC50) as measured by killing efficiency of KLK3 peptide-loaded T2 cells by TCR-transfected T cells. Constitutively luciferase-expressing T2 cells were loaded with titrated amounts of isotopologues of KLK3 peptide and then co-cultivated with CD8⁺ T cells transfected with specific TCRs. Killing was analyzed by measuring luciferase activity in the supernatant which is released by dying T2 cells. T2 cells loaded with the irrelevant NYESO peptide served as negative control and a TCR specific for NYESO peptide served as positive control as indicated in the plots. X-symbols represent the absolute copy numbers per cell of the respective T2-peptide-loading concentration as determined by immunoprecipitation followed by LC/MS.

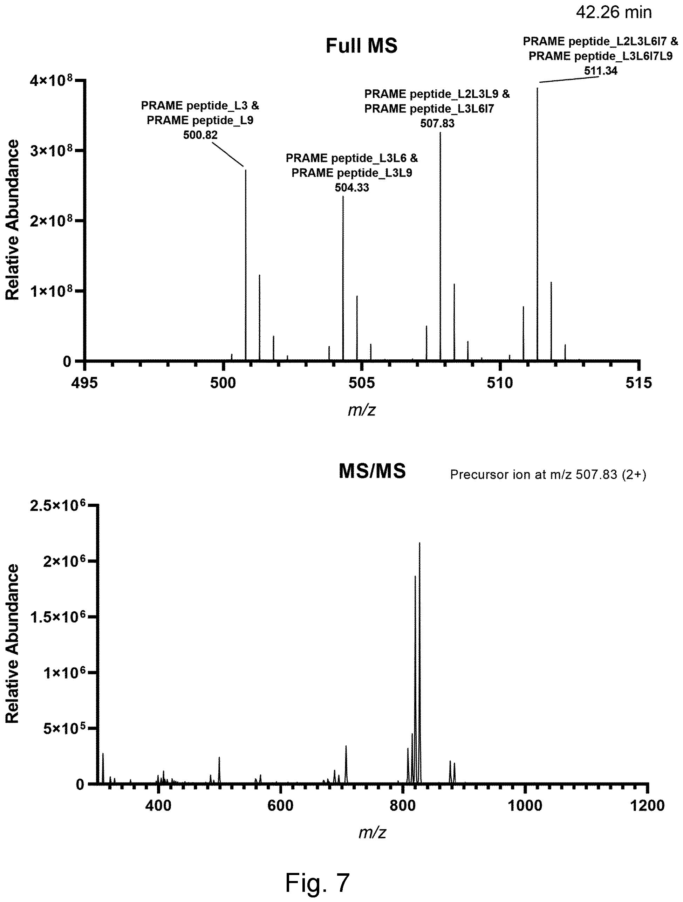

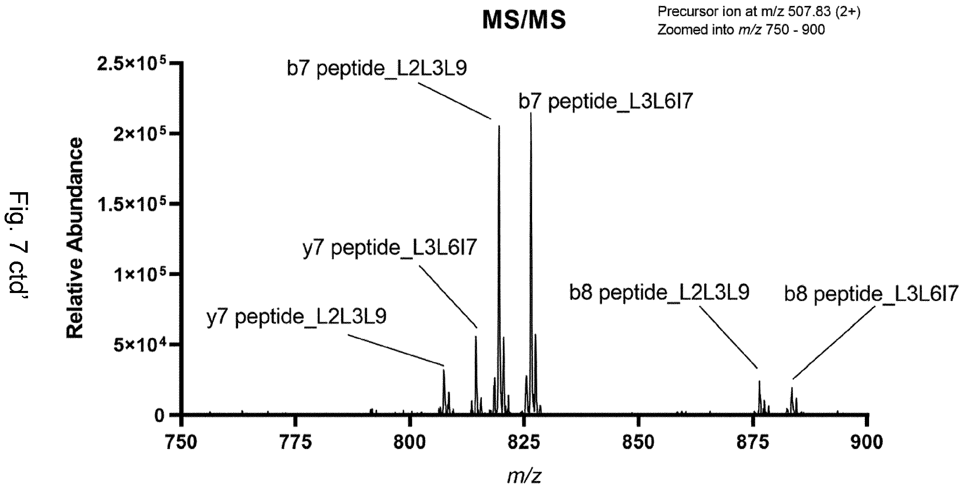

Fig. 7 shows the differentiation of selected isotopically PRAME peptide variants ("isotopologues") from each other either on the level of the precursor ion (full MS) or the resulting fragment ions (MS/MS; exemplary for 2+ precursor ions with 507.83 m/z).

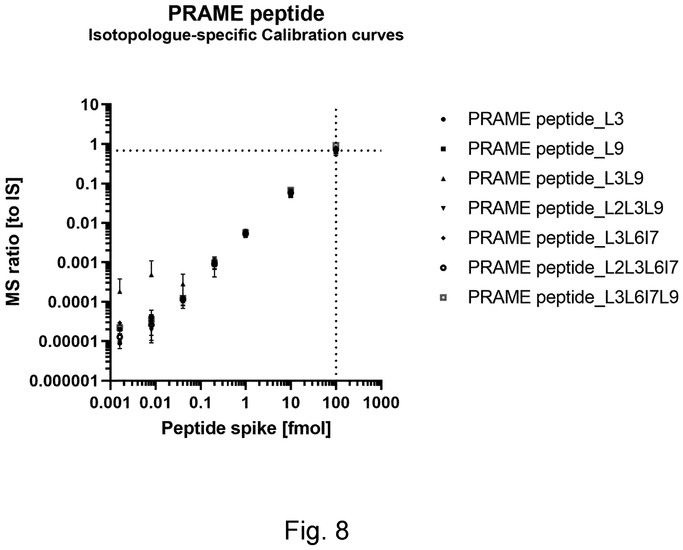

Fig. 8 shows the generation of a calibration curve which has been acquired for selected isotopically labeled variants ("isotopologues") of a given peptide (in this example, derived from PRAME (SLLQHLIGL), n = 7). The different variants have been identified in the mass spectrometry readout.
   Based on such calibration curve, MS signals measured in a given experiment can be converted into concentrations of the respective isotopically labeled variants. The underlying methods are described, *inter alia,* in WO2016107740A1, the content of which is incorporated herein by reference for enablement purposes only.

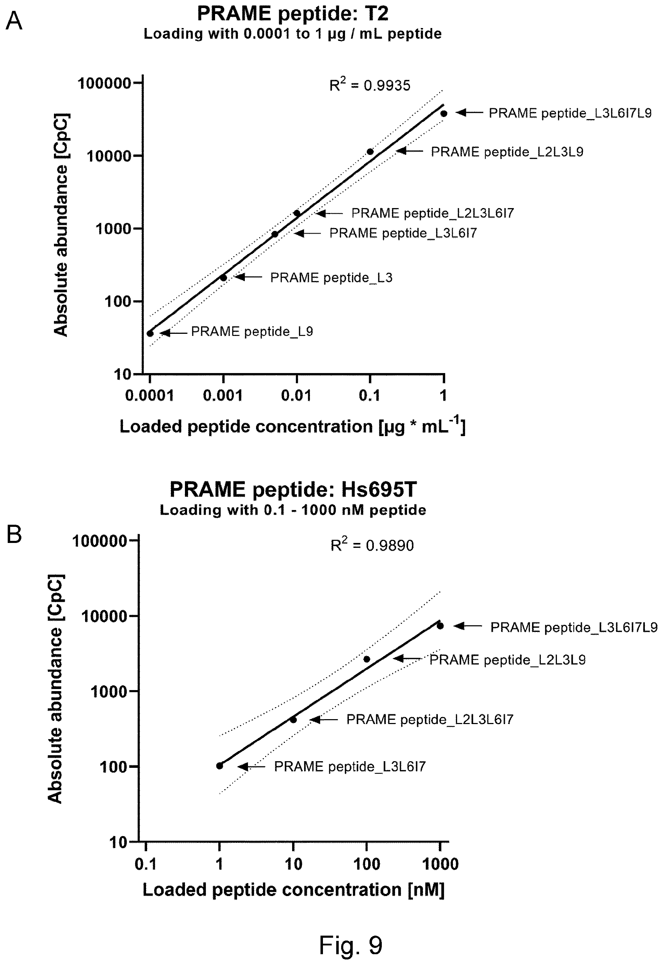

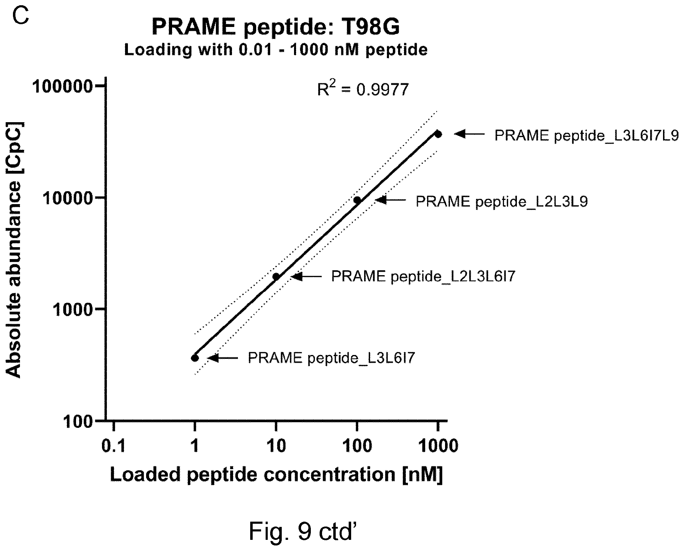

Fig. 9 shows a titration curve between (a) peptide of interest (differently labeled peptide variants in different concentrations) and (b) resulting MS signal obtained for the peptide of interest, normalized to cell count using T2 cells (Fig. 9A), Hs695T cells (Fig. 9B), or T98G cells (Fig. 9C).

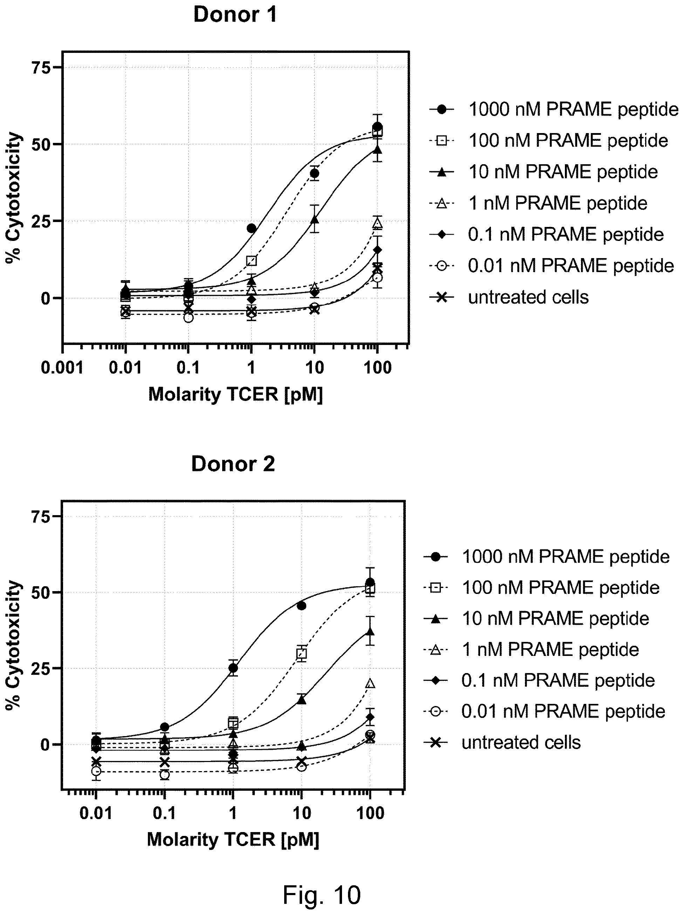

Fig. 10 shows the functional analysis of PRAME peptide loading by killing efficiency of PRAME peptide-loaded T98G cells by peripheral blood mononuclear cells (PBMCs) in the presence of a titrated PRAME-specific soluble T cell receptor ("TCER"), as disclosed in PCT/EP2020/050936, the content of which is incorporated herein by reference for enablement purposes. T98G cells were loaded with titrated amounts of isotopologues of PRAME peptide and then co-cultivated with PBMCs of two different donors and titration of PRAME-specific TCER. Cytotoxicity was analyzed by measuring lactate dehydrogenase (LDH) level in the supernatant which is released by dying T98G cells.

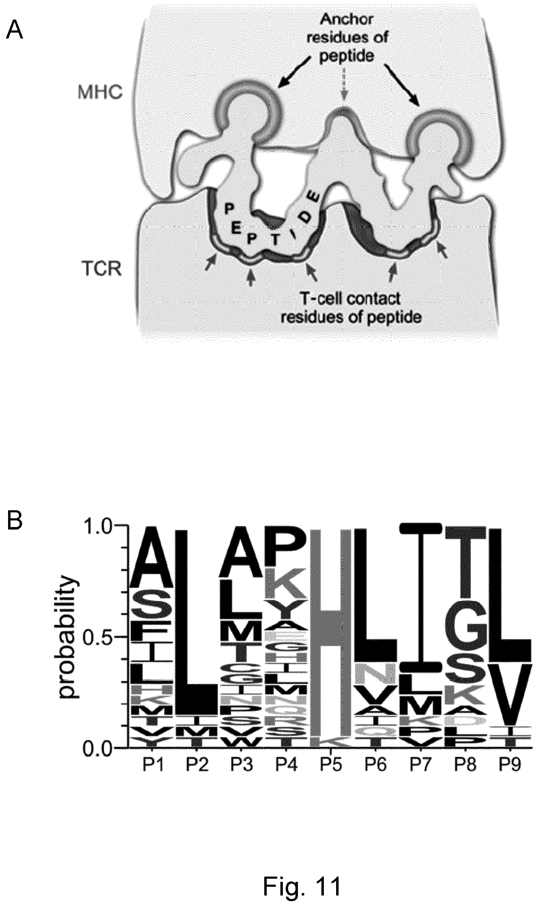

Fig. 11A illustrates, in exemplary fashion, the peptide binding groove of an MHC class I molecule formed by the α1 and α2 domain, with the so-called anchor residues of the binding peptide.
Fig. 11B shows a sequence logo of a non-specified HLA class I allotype, demonstrating the amino acid preferences at the different positions including the anchor residues P2 and P9.

### Detailed Description of the Invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a", "an", and "the" include singular and/or plural referents unless the context clearly dictates otherwise. It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

Furthermore, the content of the prior art documents referred to herein is incorporated by reference. This refers, particularly, for prior art documents that disclose standard or routine methods. In that case, the incorporation by reference has mainly the purpose to provide sufficient enabling disclosure, and avoid lengthy repetitions.

According to a first aspect of the invention, a method of characterizing the binding characteristics between a peptide of interest and MHC molecules of a given cell type is provided, the method comprising the steps of:
a) Providing two or more cells characterized by displaying, on their surface, MHC molecules,
b) dispensing the two or more cells in two or more vessels, so that each vessel comprises one or more cells
c) adding (="loading"), to the different vessels, different variants of a peptide of interest, wherein the variants of said peptide are labeled and have the same amino acid sequence, yet differ from one another in
   (i) the type of labeling, and
   (ii) their concentration
   and exposing the cells thereto so as to form, in the different vessels, peptide-MHC complexes on the surface of the cells
d) isolating the thus formed peptide-MHC complexes and
e) determining the concentration of the different peptide-MHC complexes formed in step c.

The "loading" process involves adding one or more peptides of interest capable of binding to MHC to the medium surrounding the cells.

In one embodiment, such added peptides compete for binding to the MHC with the peptides already bound thereto. If present in excess, based on the dissociation equilibrium, the added peptides will substantially replace the peptides already bound by the MHC.

In another embodiment, cells are used which comprise functionally "empty" MHC, as is described elsewhere herein. Such functionally "empty" MHCs are hence capable of directly binding the peptides that are added ("loaded") to the surrounding medium.

Herein, the term "variants of the peptide of interest" is used synonymously with the term "peptide variants".

As used herein, the term "MHC molecules" relates to class of proteins displayed on cells of vertebrates, which play a role in the cell-based immune system. Generally speaking, MHCs present peptides on their surface which are then identified by the immune system as self or non-self.

In humans, for example, three types of MHC are described, i.e., MHC class I (class Ia with *inter alia* haplotypes HLA-A, HLA-B, HLA-C; and class Ib with *inter alia* haplotypes HLA-E, HLA-F, HLA-G), MHC class II (with *inter alia* haplotypes HLA-DM, -DO, -DP, -DQ, -DR).

| | **MHC class I** | **MHC class II** |
|---|---|---|
| Molecular structure | α1, α2, α3 + β2 microglobulin | β1,β2 + α1, α2 |
| Cell type | All somatic cells | *Antigen-presenting cells* (APC) |
| Interaction with | CD8⁺ cytotoxic T cells | CD4⁺ T-helper cells |
| Typical peptide length | 8 -10 AA | 13 - 25 AA |
| Typical peptide origin | Intracellular peptides that underwent antigen processing | Exogenous peptides |

In mice, for example, at least two types of MHC are described, i.e., MHC Class I (class Ia with *inter alia* haplotypes H-2K, H-2D, H-2L, and class 1b with *inter alia* haplotypes Qa-2, Qa-1) and MHC Class II (with *inter alia* haplotypes I-A, I-E)

According to one embodiment, the MHC molecule is MHC class I

Heterodimeric MHC class I molecules are composed of a polymorphic heavy α-subunit encoded within the MHC gene cluster and a small invariant beta-2-microglobulin (β2m) subunit whose gene is located outside of the MHC locus on chromosome 15. The polymorphic α chain encompasses an N-terminal extracellular region composed by three domains, α1, α2, and α3, a transmembrane helix accomplishing cell surface attachment of the MHC molecule, and a short cytoplasmic tail. Two domains, α1 and α2, form a peptide-binding groove between two long α-helices, whereas the floor of the groove is formed by eight β-strands. The Immunoglobulin-like domain α3 is involved in the interaction with the CD8 co-receptor. The invariant β2m provides stability of the complex and participates in recognition of the peptide-MHC class I complex by CD8 co-receptors. β2m is non-covalently bound to the α-subunit. It is held by several pockets on the floor of the peptide-binding groove. Amino acid (AA) side chains that vary widely between different human HLA alleles fill up the central and widest portion of the binding groove, while conserved side chains are clustered at the narrower ends of the groove. The polymorphic amino acid residues authoritatively define the biochemical properties of peptides which can be bound by the respective HLA molecule (Boniface and Davis, 1995; Falk et al., 1991; Goldberg and Rizzo, 2015a; Rammensee et al., 1995).

In humans, the MHC class I gene cluster is characterized by polymorphism and polygenicity. Each chromosome encodes one HLA-A, -B, and -C allele together constituting the HLA class I haplotype. Consequently, up to six different classical HLA class I molecules can be expressed on the surface of an individual's cells; an exemplary combination of HLA-A, -B, and -C allotypes is given in the table below. In In June 2021, the IPD-IMGT/HLA Database (release 3.44.1, 2021-06-11) comprised a total of 6,766 HLA-A alleles (4,064 proteins), 7,967 HLA-B alleles (4,962 proteins), and 6,621 HLA-C alleles (3,831 proteins) (Robinson et al., 2015).

| HLA-A | HLA-B | HLA-C |
|---|---|---|
| A*02:01 | B*40:02 | C*03:04 |
| A*24:02 | B*52:01 | C*12:02 |

In multifactorial disease development, genetic predisposition represents a common element enclosing, *inter alia,* the composition of an individual's HLA alleles. Autoimmune disorders such as ankylosing spondylitis (HLA-B*27), celiac disease (HLA-DQA1*05:01-DQB1*02:01 or HLA-DQA1*03:01-DQB1*03:02), narcolepsy (HLA-DQB1*06:02), or type 1 diabetes (HLA-DRB1*04:01-DQB1*03:02) have a long history of HLA association (Caillat-Zucman, 2009). Moreover, it has become evident that specific HLA allotypes have an influence on the risk of contagion as well as the course of infections e.g. with the human immunodeficiency virus or malaria parasites (Hill et al., 1991; The International HIV Controllers Study et al., 2010; Trachtenberg et al., 2003). Besides that, the individual HLA genotype shapes the response to cancer immunotherapy: while maximal heterozygosity of HLA-A, -B, and -C alleles appears to favor the response to checkpoint blockade, HLA-B*15:01 has been suggested to impair neo-antigen-directed CTL responses (Chowell et al., 2018).

MHC molecules are tissue antigens that allow the immune system to bind to, recognize, and tolerate itself (autorecognition). MHC molecules also function as chaperones for intracellular peptides that are complexed with MHC heterodimers and presented to T cells as potential foreign antigens (Felix and Allen, 2007; Stern and Wiley, 1994).

MHC molecules interact with TCRs and different co-receptors to optimize binding conditions for the TCR-antigen interaction, in terms of antigen binding affinity and specificity, and signal transduction effectiveness (Boniface and Davis, 1995; Gao et al., 2000; Lustgarten et al., 1991).

Essentially, the MHC-peptide complex is a complex of auto-antigen/allo-antigen. Upon binding, T cells should in principle tolerate the auto-antigen, but activate when exposed to the allo-antigen. Disease states (especially autoimmunity) occur when this principle is disrupted (Basu et al., 2001; Felix and Allen, 2007; Whitelegg et al., 2005).

On MHC class I, a cell normally presents cytosolic peptides, mostly self-peptides derived from protein turnover and defective ribosomal products (Goldberg and Rizzo, 2015b; Schwanhausser et al., 2011, 2013; Yewdell, 2003; Yewdell et al., 1996). These peptides typically have an extended conformation and oftentimes a length of 8 to 12 amino acids residues, but accommodation of slightly longer versions is feasible as well (Guo et al., 1992; Madden et al., 1993; Rammensee, 1995). During infection with intracellular pathogens including viruses and microorganisms as well as in the course of cancerous transformation, proteins of foreign origin or associated with malignant transformation are also degraded in the proteasome, loaded onto MHC class I molecules, and further displayed on the cell surface (Goldberg and Rizzo, 2015b; Madden et al., 1993; Urban and Schreiber, 1992). Moreover, a phenomenon designated as cross-presentation accomplishes loading of extracellular antigens on MHC class I enabling activation of naive CTLs by dendritic cells (DCs) (Rock and Shen, 2005). T cells can detect a peptide displayed at 0.1%-1% of the MHC molecules and still evoke an immune reaction (Davenport et al., 2018; Sharma and Kranz, 2016; Siller-Farfan and Dushek, 2018; van der Merwe and Dushek, 2011).

According to several embodiments, the peptide of interest has a length of between 8 and 15 amino acid residues.

Such peptides are typically bound by MHC class I molecules, like e.g. HLA-A or HLA-B allotypes.

These MHC class I molecules have a peptide binding groove in their α1 and α2 domains (see Fig. 11A), in which the peptides to be displayed ate immobilized *via* so-called anchoring residues. Depending on the HLA allotype, the respective peptide is immobilized *via* two, three, or four anchoring residues. Depending on the HLA allotype one differentiates between main anchors and side anchors.

In the following table, amino acid preferences of a binding 9-mer peptide at the respective anchoring positions are shown (main anchors in bold, side anchors in italics) for selected HLA allotypes. See also Fig. 11 B, which shows a so-called sequence logo of a non-specified HLA allotype, demonstrating the preferences at the different positions including P2 and P9.

| **HLA allotype** | **P1** | **P2** | **P3** | **P4** | **P5** | **P6** | **P7** | **P8** | **P9** | **Main anchoring residues** | **Side anchoring residues** |
|---|---|---|---|---|---|---|---|---|---|---|---|
| **A*01:01** | | **T** | **D** | | | | | | **Y** | P2, P3, P9 | |
| | | **S** | **E** | | | | | | | | |
| | | **L** | | | | | | | | | |
| **A*02:01** | | **L** | | *E* | | *L* | | | **V** | P2, P9 | P4, P6 |
| | | | | *D* | | *V* | | | **L** | | |
| | | | | *P* | | *I* | | | | | |
| **A*03:01** | *R* | **V** | | | | | *L* | | **K** | P2, P9 | P1, P7 |
| | *K* | **L** | | | | | *I* | | | | |
| | *A* | **I** | | | | | *V* | | | | |
| | | **T** | | | | | | | | | |
| **A*24:02** | | **Y** | | | | | | | **F** | P2, P9 | |
| | | | | | | | | | **L** | | |
| | | | | | | | | | **I** | | |
| **B*07:02** | *R* | **P** | *R* | | | | | | **L** | P2, P9 | P1, P3 |
| | *S* | | *A* | | | | | | | | |
| | *A* | | | | | | | | | | |
| **B*08:01** | | *L* | *K* | | **K** | | | | **L** | P5, P9 | P2, P3 |
| | | *P* | *L* | | **R** | | | | | | |
| | | *I* | *R* | | | | | | | | |
| | | *A* | | | | | | | | | |
| **B*44:02** | *E* | **E** | | | | | | | **W** | P2, P9 | P1 |
| | *A* | | | | | | | | **F** | | |
| | *S* | | | | | | | | **Y** | | |
| **B*44:03** | *E* | **E** | | | | | | | **Y** | P2, P9 | P1 |
| | *A* | | | | | | | | **F** | | |
| | *S* | | | | | | | | **W** | | |
| | | | | | | | | | **L** | | |

For binding peptides other than 9-mers, amino acids are inserted or removed at P5 to represent the motif accordingly. In the following table, amino acid preferences at anchor and side anchor positions are exemplarily shown for HLA-A*02:01 and peptides of 8 to 13 AAs length.

| | | | | | | | | | | | | | |
|---|---|---|---|---|---|---|---|---|---|---|---|---|---|
| **8-mer** | **P1** | **P2** | **P3** | **P4** | **P5** | **P6** | **P7** | **P8** | | | | | |
| | | **L** | | *E* | *L* | | | **V** | | | | | |
| | | | | *D* | *V* | | | **L** | | | | | |
| | | | | *P* | *I* | | | | | | | | |
| **9-mer** | **P1** | **P2** | **P3** | **P4** | **P5** | **P6** | **P7** | **P8** | **P9** | | | | |
| | | **L** | | *E* | | *L* | | | **V** | | | | |
| | | | | *D* | | *V* | | | **L** | | | | |
| | | | | *P* | | *I* | | | | | | | |
| **10-mer** | **P1** | **P2** | **P3** | **P4** | **P5** | **P6** | **P7** | **P8** | **P9** | **P10** | | | |
| | | **L** | | *E* | | | *L* | | | **V** | | | |
| | | | | *D* | | | *V* | | | **L** | | | |
| | | | | *P* | | | *I* | | | | | | |
| **11-mer** | **P1** | **P2** | **P3** | **P4** | **P5** | **P6** | **P7** | **P8** | **P9** | **P10** | **P11** | | |
| | | **L** | | *E* | | | | *L* | | | **V** | | |
| | | | | *D* | | | | *V* | | | **L** | | |
| | | | | *P* | | | | *I* | | | | | |
| **12-mer** | **P1** | **P2** | **P3** | **P4** | **P5** | **P6** | **P7** | **P8** | **P9** | **P10** | **P11** | **P12** | |
| | | **L** | | *E* | | | | | *L* | | | **V** | |
| | | | | *D* | | | | | *V* | | | **L** | |
| | | | | *P* | | | | | *I* | | | | |
| **13-mer** | **P1** | **P2** | **P3** | **P4** | **P5** | **P6** | **P7** | **P8** | **P9** | **P10** | **P11** | **P12** | **P13** |
| | | **L** | | *E* | | | | | | *L* | | | **V** |
| | | | | *D* | | | | | | *V* | | | **L** |
| | | | | *P* | | | | | | *I* | | | |

On the basis of the above, according to several embodiments, the peptide of interest has the following sequence motif XₘA₁XₙA₂Xₒ, wherein
- X is any proteinogenic amino acid
- A₁ is an amino acid selected from the group consisting of T, A, E, I, L, P, S, V, Y
- A₂ is an amino acid selected from the group consisting of Y, F, I, K, L, V, W
- m is an integer between 1 and 10
- n is 6
- o is an integer between ≥ 1 and ≤ 10, and
- m + o ≤ 7

According to further embodiments, the peptide of interest is a tumor-associated peptide (TUMAP) or a disease-associated peptide.

As the name suggests, a tumor-associated peptide (TUMAP) or a disease-associated peptide is a peptide that is found on the surface of cancerous or elsehow diseased cells, yet not on healthy cells, or is present on the surface of cancerous or elsehow diseased cells in significantly higher abundance than on healthy cells.

According to further embodiments, the variants of the peptide of interest are isotopically labeled ("isotopologues").

According to further embodiments, the isotopical labeling comprises at least one isotopically labeled amino acid.

In general, for each amino acid, isotopically labeled variants exist and can be purchased. In one embodiment, however, the amino acids A and G are never isotopically labeled.

According to further embodiments, the different variants of the peptide of interest differ from one another in the type of isotopical labeling.

Such difference can comprise, *inter alia,* the type of isotopically labeled amino acid residue and/or the total amount of isotopically labeled amino acid residues in the respective peptide.

According to one embodiment of the invention, the peptide-MHC complexes are isolated by immunoaffinity enrichment.

Methods of immunoaffinity enrichment - sometimes also called "immunoprecipitation" - are disclosed, *inter alia,* in (Caron et al., 2015) as well as in (Freudenmann et al., 2018), (Kowalewski and Stevanovic, 2013) and (Kasuga, 2013), the contents of which are incorporated herein by reference for enablement purposes only.

Commonly, HLA class I and class II peptide isolation is achieved from cell lysates. The cell suspensions are mechanically homogenized and lysed, preferably by employing non-denaturing detergents, such as NP-40, Triton X-100, CHAPS, sodium deoxycholate, or IGEPAL CA-630. Lysis buffers can contain protease inhibitors to block degradation of HLA-peptide complexes.

The cleared lysate is then for example subjected to immunoaffinity chromatography employing an MHC-binding polypeptide as discussed below. Such MHC binding polypeptide can be covalently coupled to a matrix, like e.g. sepharose or agarose resins, or non-covalently attached to Protein A or Protein G. Different commercial cross-linking technologies are available, such as CNBr-activated sepharose or AminoLink^{™} coupling resin, which employs aldehyde-activated 4% beaded agarose. Sometimes the lysate is precleared from native antibodies before immunoaffinity chromatography with Protein A or Protein G.

As an alternative to immunoaffinity enrichment of MHC molecules, (loaded) peptides may also be released from MHC molecules by mild acid elution (Freudenmann et al., 2018; Storkus et al., 1993).

According to one embodiment, the immunoaffinity enrichment is carried out using an MHC binding polypeptide.

It is in this context important to differentiate between the terms "MHC-binding polypeptide." and "pMHC-binding protein". While an MHC-binding polypeptide has specificity to MHC and binds the latter irrespective of whether it has bound a peptide or not, and irrespective of the sequence or structure of such peptide, a pMHC-binding protein binds specifically to a given peptide:MHC (pMHC) complex, depending on, *inter alia,* the sequence or structure of the peptide. Hence, an MHC-binding polypeptide can for example be used for immunoaffinity enrichment of MHCs or pMHCs, irrespective of the peptide's sequence or structure, while pMHC-binding proteins can be used as therapeutic entities to bind to a specific peptide:MHC (pMHC) complex, and evoke a physiological reaction.

According to one embodiment, the immunoaffinity enrichment is carried out using an MHC-specific antibody.

To isolate and analyse MHC-presented peptides, monoclonal antibodies specific for the MHC molecule(s) of interest are commonly applied (Freudenmann et al., 2018). It is important to mention that such antibodies bind to the MHC independent of the respective peptide bound thereby. For different HLA allotypes, different antibodies are available, although antibodies exist which bind to different HLA allotypes. An overview of such antibodies specific for human or murine MHC is given in the following table:

| **Clone** | **Specificity** | **Reference** |
|---|---|---|
| W6/32 | HLA-A, -B, -C | (Barnstable et al., 1978) |
| B1.23.2 | HLA-B, -C | (Rebai et al., 1983) |
| BB7.2 | HLA-A*02 | (Parham and Brodsky, 1981) |
| GAP-A3 | HLA-A*03 | (Berger et al., 1982) |
| ME1 | HLA-B*07, -B*27, -Bw22 | (Ellis et al., 1982) |
| Tü39 | HLA-DR, -DP, -DQ | (Maeda and Hirata, 1984) |
| B7/21 | HLA-DP | (Robbins et al., 1987; Royston et al., 1981) |
| L243 | HLA-DR | (Lampson and Levy, 1980) |
| LB3.1 | HLA-DR | (Gorga et al., 1986) |
| Spv-L3 | HLA-DQ | (Spits et al., 1983) |
| IVD-12 | HLA-DQ | (Kolstad et al., 1987) |
| Y-3 | H-2K^{b} | (Hämmerling et al., 1982; Jones and Janeway, 1981) |
| B8-24-3 | H-2K^{b} | (Kohler et al., 1981) |
| 20-8-4S | H-2K^{b} | (Ozato and Sachs, 1981) |
| 28-8-6S | H-2K^{b} | (Ozato and Sachs, 1981) |
| 5F1 | H-2K^{b} | (Hämmerling et al., 1982; Sherman and Randolph, 1981) |
| B22-249.R1 | H2-D^{b} | (Lemke et al., 1979) |
| 28-14-8S | H-2D^{b} | (Ozato and Sachs, 1981) |
| 27-11-13S | H-2D^{b} | (Ozato and Sachs, 1981) |
| M5/114 | la | (Bhattacharya et al., 1981) |

Further suitable antibodies are disclosed, *inter alia,* in (Sidney et al., 2013) the content of which is incorporated herein by reference for enablement purposes only.

According to one embodiment, after isolation of the peptide-MHC complexes, the peptides are eluted from the MHCs.

As used herein, the term "eluted" relates to a process in which the peptides are released from the peptide-MHC complexes. The term "eluate" designates the medium that comprises eluted peptides.

Elution of HLA complexes can for example be achieved either through treatment with a strong acid, such as 0.1-0.2% TFA (trifluoroacetic acid), 10% acetic acid or with 0.1-0.2 N acetic acid followed by heat denaturation. Both approaches lead to denaturation of the MHC molecule, and the release of the peptide bound.

As an alternative to immunoaffinity enrichment and subsequent peptide elution, the peptides bound by MHC can also be isolated by mild acid elution (MAE) from whole cells, to induce dissociation of the non-covalently bound β2-microglobulin and the peptide from the MHC complexes on the cell surface. Typically, a buffer like citrate phosphate buffer at moderately low pH (e.g.: pH 3.3) is used for about 1 min. MAE is supposed to isolate MHC-bound peptides with fewer purification steps, detergent-free, and without the bias linked to preferential loss of low-affinity peptides. However, contaminating peptides interacting with the cell membrane *via* hydrostatic forces may also be eluted by mild acid treatment. These could be discriminated from MHC-bound peptides by analyzing an equivalent negative control as well, possibly a β2-microglobulin-deficient cell line.

According to one embodiment, the concentration of the different peptide variants is determined in the eluate, so as to determine the concentration of the different peptide-MHC complexes formed in step c).

Due to the 1:1 stochiometry between peptides and MHC, the concentration of peptides found equals the concentration of peptide-MHC complexes formed in step c) (with the caveat that complexes could get lost during the purification process).

The concentration of the different peptide variants in the eluate can for example be determined by means of LC-MS/MS, as described, *inter alia,* in WO2016107740A1, the content of which is incorporated herein for enablement purposes only.
In one embodiment, the method comprises
(1) determining the cell count of each preparation in which cells are or have been exposed to different variants and concentrations of the peptide of interest,
(2) adding a known amount of peptide of interest, optionally bound to MHC to said preparation of step a), preferably directly after tissue homogenization ("spiking I"),
(3) isolating or purifying the formed peptide-MHC complexes,
(4) eluting the peptides from the MHC
(5) adding a known amount of the peptide of interest to be quantified to said peptide eluate ("spiking II") as an internal calibrant,
(6) performing a mass spectrometry analysis on said peptide of interest in order to generate
   (i) a signal for the efficiency of the isolation in step (3),
   (ii) a signal for the internal calibrant of step (5), and
   (iii) a signal for the total amount of peptide of interest and
(7) quantifying said MHC ligand based on a comparison of the signals as obtained in step (6) with
   (i) the cell count as obtained,
   (ii) the known amount of said peptide of interest and/or peptide-MHC complex to be quantified as added in step (2), and
   (iii) the known amount of MHC peptide ligand to be quantified as added in step (5), wherein quantifying comprises calculating a ratio between the signals of the internal calibrant of step (5) and of the peptide of interest and comparing the ratio with the calibration curve.

Optionally, quantifying further comprises the generation of a peptide-specific calibration curve based on a ratio with the internal calibrant used at the same amount, and determination of the lowest level of quantification (LLOQ) for said peptide of interest to be quantified, whereby an absolute quantification of peptide of interest on a cell is achieved if the quantified amount is above the LLOQ as determined.

According to one embodiment, the method of the invention further comprises determining the amount of at least one type of MHC molecules in said preparation of step a). Methods to determine amount of at least one type of MHC molecule are disclosed, *inter alia,* in DE1020211051428 and the PCT application claiming it's priority.

According to one embodiment of the method of the invention, a ratio between
(i) the concentrations of the peptide of interest to which the cells are exposed in step b) and
(ii) the concentrations of different peptide-MHC complexes formed in step c) is determined.

According to one embodiment of the invention, for each peptide variant, the cell count of the cells exposed thereto is determined.

In order to do this, different approaches are available, like counting cells or cell nuclei by means of microscopy and/or optical image processing, photometric DNA-determination, fluorimetric DNA-determination quantitative PCR, quantitative determination of histones by means of LC-MS, or automatic cell counting (with e.g. the CASY instrument).

According to one embodiment of the invention, the calculated ratio is peptide concentration to which the cells are exposed in step b) (µg mL⁻¹ or nM) vs. copies of peptide in pMHC complexes per cell.

Such ratios can be determined on the basis of
(i) the known concentrations of peptide of interest to which the cells are exposed in step b),
(ii) the concentrations of the different peptide-MHC complexes formed in step c), as determined in step d), and optionally
(iii) the number of cells which were exposed to the respective peptide variants

On the basis of the different ratios, a calibration curve or formula can be established. As a result, it can be predicted, if cells are exposed to a given concentration of peptide of interest, how many peptide MHC complexes will form, either in general or per cell.

This again is extremely helpful in experiments where cells that have been loaded with a peptide of interest are exposed to one or more pMHC-binding entities, to for example characterize
a) the potency of such pMHC-binding entities in binding assay or a biological assay
b) the affinity or specificity of such pMHC-binding entities to the respective peptide-MHC complexes

According to one embodiment of the invention, the concentration of the different peptide variants is determined on the one or more by means of at least one method selected from the group consisting of
- mass spectrometry (MS)
- tandem mass spectrometry (MS/MS)
- liquid chromatography coupled with mass spectrometry (LC-MS, LC-MS/MS

Methods of determining the concentration of peptides formerly bound to MHC by mass spectrometry (MS) are disclosed, *inter alia,* in WOWO2016107740, the content of which is incorporated herein by reference, and in (Freudenmann et al., 2018), the content of which is incorporated herein by reference. Among these, liquid chromatography-coupled tandem mass spectrometry (LC-MS/MS) is a particularly suitable approach.

Peptide sequencing by liquid chromatography-coupled tandem mass spectrometry (LC-MS/MS) is achieved by prefractionation of complex peptide solutions, followed by MS. Typically, MS1 survey spectra are acquired and abundant peptides are selected for fragmentation yielding MS2 spectra. Prefractionation is often performed by a chromatography step, like e.g. reversed-phase or SCX (strong cation exchange) chromatographic separation.

MS sequencing is frequently accomplished by using CID or beam-type higher-energy CID (HCD). These methods produce peptide fragment ions that can be used in automated database search strategies or *de novo* analysis to identify peptide sequences. However, these methods have been optimized for tryptic peptides and are not ideal for HLA ligands, as generated spectra often lack sufficient fragment information for confident identification. Therefore, the use of hybrid fragmentation methods such as electron-transfer/higher-energy-induced dissociation (EThCD) has been proposed. For a comprehensive review about MS acquisition strategies, see (Caron et al., 2015) or (Schumacher et al., 2017), the contents of which are incorporated herein by reference for enablement purposes

According to one embodiment of the invention, the peptide that forms part of the peptide-MHC complex is a peptide that is not presented by an established cell line.

This applies, for example, for peptides derived from KLK3. So far, no cell lines have been described which express KLK3 and/or display MHC-restricted peptides derived from KLK3 on their surfaces. One such KLK3-derived MHC-restricted peptide is shown in SEQ ID NO 1.

For such peptides, which may represent valuable targets for e.g. cancer therapy (e.g., by means of suitable therapeutic entities, like e.g. adoptive T cells, soluble T cell receptors (TCRs) or TCR mimetic antibodies, it may be difficult to develop suitable *in vitro* systems or cell-based assays to investigate potency of such therapeutic entity candidates. For these peptides, the method according to the invention allows to artificially establish cells that present the peptides, and to then investigate responses upon exposure to respective therapeutic entities, and also to establish dose-response curves.

However, the method according to the invention can also be used for peptides that actually are presented by established cell lines. This would for example be the case for PRAME, peptides

To study the activity and potency of therapeutic entities that can bind to peptide-MHC complexes *in vitro,* it is inevitable to have a suitable model system at one's disposal presenting the target peptide(s) on its MHC molecules. Ideally, the presentation level, i.e. the number of peptide copies per target cell, is comparable to that observed on native patient tissue representing the indication in which the investigative drug product finally has to be active and safe.

Similarly, it may also be of interest to study off-target effects in cells negative for the target peptide while presenting (potential) off-target peptides (in a defined amount) (Liu et al., 2020). However, it is not always feasible to establish a suitable model system, e.g. a cell line, presenting the peptide of interest at all or at the desired copy number range.

According to one embodiment of the invention, the two or more cells characterized by displaying, on their surface, MHC molecules, are deficient in peptide antigen processing and/or peptide antigen presentation.

Such cell lines are (almost) devoid of endogenous MHC presentation of peptides.

According to one embodiment of the invention, the cells' deficiency in peptide antigen processing and/or presentation is a caused by deficiency of the transporter associated with antigen processing (TAP).

Transporter associated with antigen processing (TAP) protein complex belongs to the ATP-binding-cassette transporter family. It delivers cytosolic peptides into the endoplasmic reticulum (ER), where they bind to nascent MHC class I molecules.

The TAP structure is formed of two proteins: TAP-1 (NCBI gene: 6890) and TAP-2 (NCBI gene: 6891), which have one hydrophobic region and one ATP-binding region each. They assemble into a heterodimer, which results in a four-domain transporter.

Such cells represent prime candidates for being externally loaded with MHC-binding peptides of interest. Externally added synthetic peptides facilitate MHC class I assembly and/or bind to and stabilize empty MHC class I-β2m heterodimers (Lewis et al., 1996; Liu et al., 2020; Ljunggren et al., 1991; Salter and Cresswell, 1986; Townsend et al., 1989). These may either be naturally expressed (empty) MHC molecules or such being introduced by transfection into MHC-deficient cells (DeMars et al., 1984; Lewis et al., 1996; Riberdy and Cresswell, 1992)

According to one embodiment of the invention, the cell's deficiency in peptide antigen processing and/or presentation results in the expression of functionally "empty" class I MHC on their cell surface.

As used herein, the term "empty" MHC means that the cells presents MHC on its surface which fail to come with a bound T-cell epitope peptide. Such functionally "empty" MHCs are hence capable of binding respective peptides that are added ("loaded") to the surrounding medium.

According to embodiments of the invention, the cell is selected from the group consisting of
- T2 (174xCEM.T2)
- RMA-S
- B-LCL 721.174 or B-LCL 721.180
- C1R-T134K

T2 is a lymphoma-derived cell line that express low amounts of HLA-A2 on the cell surface due to TAP deficiency and can only present exogenous peptides. Binding of exogenous peptides to HLA-A2 stabilizes the HLA-A2-peptide complexes and can be detected using immunofluorescence staining.

RMA-S mutant cell lines have a defect in class-I assembly and express markedly reduced levels of class-I molecules at the cell surface.

Characteristics of these cell lines that can be used in the context of the present invention are shown in the following table:

| **Cell line** | **Malfunction of antigen processing machinery** | **Reference** |
|---|---|---|
| B-LCL 721.174 B-LCL 721.180 | Homozygous deletion of functional class II genes (including TAP) | (DeMars et al., 1984; Erlich et al., 1986) |
| (human B lymphoblastoid cell line) | Severely reduced HLA class I and II expression | |
| .174xCEM.T2 ('T2'; hybrid of human B-LCL 721.174 and human T-LCL CEMR.3) | Homozygous deletion of functional class II genes (including TAP) | (Erlich et al., 1986; Riberdy and Cresswell, 1992; Salter and Cresswell, 1986; Salter et al., 1985) |
| | Normal HLA class I heavy chains and β2m, defect in HLA assembly / empty HLA molecules | |
| C1R-T134K (human B lymphoblastoid cell line transfected with HLA-A2.1 T134K) | C1R: Low expression of HLA class I molecules, (deletion of HLA-A*03, -Cw3, - Bw62 / impaired transcription of HLA-A*02 / impaired translation of HLA-B*35 / defect in HLA-Cw4 assembly | (Lewis et al., 1996; Storkus et al., 1987; Zemmour, 1996; Zemmour et al., 1992) |
| | HLA-A2.1 T134K: mutation in α2 domain disables interaction with TAP. Defect in HLA assembly / empty HLA molecules | |
| RMA-S (murine T-cell lymphoma) | TAP deficiency | (Ljunggren and Karre, 1985; Ljunggren et al., 1991; Townsend et al., 1989) |
| | Reduced MHC class I expression, defect in MHC class I assembly / empty class I molecules | |

Besides using cell lines with a malfunction of the antigen processing machinery, it is further to possible to externally load any other cell line (e.g. NCIH1755, T98G, Hs695T) positive for the HLA allotype of interest - either naturally or introduced by transfection - with peptide(s) of interest.

In such case, the loading peptide is provided in such way that it competes with the different peptides already bound by the cells' MHCs for binding thereto.

According to one embodiment of the invention, the method further comprises subjecting at least a part of the cells that have been exposed to the peptide of interest to an assay in which the interaction of a pMHC-binding protein or a pMHC-binding cell to the thus formed peptide-MHC complexes is characterized.

It is in this context important to differentiate between the terms "MHC-binding polypeptide." and "pMHC-binding protein". While an MHC-binding polypeptide has specificity to MHC and binds the latter irrespective of whether it has bound a peptide or not, and irrespective of the sequence or structure of such peptide, a pMHC-binding protein binds specifically to a given peptide:MHC (pMHC) complex, depending on, *inter alia,* the sequence or structure of the peptide. Hence, an MHC-binding polypeptide can for example be used for immunoaffinity enrichment of MHCs or pMHCs, irrespective of the peptide's sequence or structure, while pMHC-binding proteins can be used as therapeutic entities to bind to a specific peptide:MHC (pMHC) complexes, and evoke a physiological reaction.

According to one embodiment of the invention, the method further comprises the determination of a dosage-response relationship related to the interaction between the pMHC-binding protein or the pMHC-binding cell and the pMHC.

According to one embodiment of the invention, the assay is a biological assay.

Such biological assay is for example a functional assay like e.g. a cytokine release assay.

One such assay is ELISPOT. In such assay, T cells are cultured together with peptide-loaded antigen-presenting cells as produced according to the invention. In case the T cells comprise a matching T-cell receptor that is capable of binding to the peptide-MHC complex of the peptide-loaded antigen-presenting cells, the T cells will for example release interferon gamma. The latter is then quantified e.g. by means of an anti-interferon antibody, which is for example provided as a coating of the respective reaction well. Such approach is sometimes called ELISPOT (enzyme liked immunospot). In such way, a dose-response curve can be established between quantity of peptide of interest and cytokine release. The ELISPOT assay has also been described for the detection of tumor necrosis factor alpha, interleukin-(IL-)4 IL-5, IL-6, IL-10, IL-12, granulocyte-macrophage colony-stimulating factor, and even granzyme B-secreting lymphocytes. See (Bercovici et al., 2000), the content of which is incorporated herein by reference for enablement purposes, for a review.

As an alternative, a soluble, bifunctional T-cell receptor can be used which has an anti-CD3 antibody fused thereto. The T-cell receptor is incubated with the cells, and unbound T-cell receptor is removed by washing, T cells are then added and engaged by bound bifunctional T-cell receptor, so that they release cytokine, which is then quantified.

Another such assay is flow cytometric analyses of intracellular cytokines. This assay measures the cytokine content in culture supernatants. When T cells are treated with inhibitors of secretion such as monensin or brefeldin A, they accumulate cytokines within their cytoplasm upon antigen activation. After fixation and permeabilization of the lymphocytes, intracellular cytokines can be quantified by cytometry. This technique allows the determination of the cytokines produced, the type of cells that produce these cytokines, and the quantity of cytokine produced per cell. See again (Bercovici et al., 2000), the content of which is incorporated herein by reference for enablement purposes, for a review.

Another such biological assay is a cytotoxicity assay, which involves the measurement of target cell lysis caused by cytotoxic T cells (CTLs). The gold standard for CTL lysis has been the ⁵¹Cr-release assay in which ⁵¹Cr is added to target cells and the amount of ⁵¹Cr released by lysed cells is measured. Detection of mouse or human CTL activity usually relies on cytotoxicity assays where peripheral blood mononuclear cells (PBMCs) or spleen cells are stimulated with their cognate ligand (usually an MHC class I-restricted peptide displayed on the surface of a given cell type) and expanded by addition of IL-2 over 1 week, and then tested for their ability to lyse ⁵¹Cr-loaded cells.

Another such assay is a cytotoxicity assay, which involves the measurement of target cell lysis caused by CTLs. CTL lysis efficiency is quantified by measuring lactate dehydrogenase (LDH) levels in the supernatant released from dying or apoptotic cells.

Another such assay is a cytotoxicity assay, which involves the measurement of target cell lysis caused by CTLs. Therefore, target cells are genetically modified to constitutively express luciferase. Upon target cell lysis, luciferase activity can be measured in the supernatant by adding specific substrate and measuring the chemiluminescent signal.

According to one embodiment of the invention, the biological assay is a cytokine release assay.

According to one embodiment of the invention, the assay is an *in vitro* assay.

According to one embodiment of the invention, the *in vitro* assay is a surface plasmon resonance assay.

Surface plasmon resonance (SPR) is the resonant oscillation of conduction electrons at the interface between negative and positive permittivity material stimulated by incident light. SPR is the basis of many standard tools for measuring adsorption of material onto planar metal (typically gold or silver) surfaces or onto the surface of metal nanoparticles. SPR-based biosensors can be used in determination of active concentration as well as characterization of molecular interactions in terms of both affinity and chemical kinetics. One typical SPR-based biosensor type is Biacore.

According to further embodiments of the invention, the *in vitro* assay is one of
- LDH cytotoxicity assay
- luciferase cytotoxicity assay
- ⁵¹CR release assay

LDH cytotoxicity assays provide a simple, reliable method for quantifying cellular cytotoxicity. Lactate dehydrogenase (LDH) is a cytosolic enzyme present in many different cell types. Plasma membrane damage releases LDH into the cell culture media. Extracellular LDH in the media can be quantified by a coupled enzymatic reaction in which LDH catalyzes the conversion of lactate to pyruvate via NAD+ reduction to NADH. Diaphorase then uses NADH to reduce a tetrazolium salt (INT) to a red formazan product that can be measured at 490nm. The level of formazan formation is directly proportional to the amount of LDH released into the medium, which is indicative of cytotoxicity.

Luciferase cytotoxicity assays measures the relative number of dead cells in cell populations. The assays measure the extracellular activity of a distinct intracellular protease activity (dead-cell protease) when the protease is released from membrane-compromised cells. A luminogenic cell-impermeant peptide substrate (e.g. AAF-aminoluciferin) is used to measure dead-cell protease activity. The liberated aminoluciferin product is measured as luminescence generated by a Luciferase provided in the assay reagent. The AAF-aminoluciferin substrate cannot cross the intact membrane of viable cells and does not generate any appreciable signal from the live-cell population. The amount of luminescence directly correlates with the percentage of cells undergoing cytotoxic stress.

Chromium-51 (⁵¹Cr) release assays are commonly used for the precise and accurate quantification of cytotoxicity, particularly in the study of tumor and viral cytolysis. The assay is used to determine the number of lymphocytes produced in response to infection or drug treatment.

Target cells are labeled with ⁵¹Cr, the label is then released from the target cells by cytolysis. The label can be isolated by centrifuging the samples and collecting the supernatants. Supernatants from centrifugation can either be counted directly in a gamma counter, or mixed with scintillation cocktail in a microplate (or dried on a LumaPlate^{™}) and counted in a liquid scintillation counter.

According to embodiments of the invention, the pMHC binding protein is selected from the group consisting of:
- a T-cell receptor, or a target binding fragment thereof, or
- a TCR-mimic antibody, or a target binding fragment thereof.

T-cell receptors that are suitable for the above purpose are for example disclosed in WO2019012141A1, the content of which is incorporated herein by reference. Such TCRs comprise, *inter alia,* the TCR α and β chains, devoid of the transmembrane domains. Specificity to the respective pMHC complex is mediated by the variable domains of the TCR α and β chains, in particular by the complementarity-determining regions (CDRs) comprised therein. Further, such T-cell receptors can comprise an effector moiety, like e.g. an inflammatory cytokine, or a CD3-binding moiety, like an anti-CD3 antibody or antibody fragment. By this mechanism, T cells are redirected to the site of disease and mount a cytolytic/apoptotic attack against the target cells (Chang et al., 2016; Dao et al., 2015; He et al., 2019). Further, such T-cell receptor can comprise moieties that increase serum half like, like e.g. an Fc domain. Other T-cell receptors suitable for the above purpose are e.g. disclosed in EP3112376A1, the content of which is incorporated herein by reference.

TCR-mimic antibodies (also called TCR mimetics) are antibodies which specifically bind to a pMHC complex. TCR-mimic antibodies that are suitable for the above purpose are for example disclosed in (Chames et al., 2000; Denkberg et al., 2003; Neethling et al., 2008; Willemsen et al., 2005).

According to one embodiment of the invention, the pMHC-binding cell is a T cell.

In adoptive T-cell therapy, a patient's own T cells are isolated, optionally enriched for clones with desired specificity against the peptide of interest, expanded *in vitro,* and re-infused into the patient.

According to embodiments of the invention, the T cell is an engineered or non-engineered T-cell comprising a homologous or heterologous T-cell receptor

As used herein, the term "homologous T-cell receptor" is meant to designate the naturally occurring T-cell receptor of the respective T cell or T-cell clone.

As used herein, the term "heterologous T-cell receptor" is meant to designate a T-cell receptor which has been engineered to recognize, or naturally recognizes, the peptide of interest. Said TCR has been recombinantly introduced into a T cell. In such way, these T cells are "reprogrammed" to bind to cells at the site of disease and exert a cytolytic/apoptotic attack against these target cells.

In some embodiment, co-stimulatory molecules such as CD40 ligand are incorporated into these T cells equipped with chimeric antigen receptors (CAR) to further enhance the triggered anti-tumor immune response (Kuhn et al., 2019; Rosenberg et al., 2011).

### Examples

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

All amino acid sequences disclosed herein are shown from N-terminus to C-terminus.

### Example 1: KLK3 Peptide

### Experimental details

Prostate-specific antigen (PSA), also known as gamma-seminoprotein or kallikrein-3 (KLK3), P-30 antigen, is a glycoprotein enzyme encoded in humans by the KLK3 gene. PSA is a member of the kallikrein-related peptidase family and is secreted by the epithelial cells of the prostate gland.

PSA is produced for the ejaculate, where it liquefies semen in the seminal coagulum and allows sperm to swim freely. It is also believed to be instrumental in dissolving cervical mucus, allowing the entry of sperm into the uterus.

PSA is present in small quantities in the serum of men with healthy prostates, but is often elevated in the presence of prostate cancer or other prostate disorders. PSA is not uniquely an indicator of prostate cancer, but may also detect prostatitis or benign prostatic hyperplasia.

MHC-restricted peptides that are derived from KLK3 have for example been disclosed in US 1044923 8B2, the content of which is incorporated herein by reference.

### 1. Isotopologue Selection & Synthesis

A total of 7 isotopologues were selected of which six were used for T2 loading experiments (spike I) and one which served as the internal standard (spike II) for downstream LC/MS analysis (Table 1). Labeling positions within the respective peptide sequence were chosen based on two criteria of which at least one had to be fulfilled for the unambiguous detection and quantification:
1. Unique precursor mass within the selected set
2. Shared precursor mass but unique fragment ions / transitions compared to other isobaric peptides/isotopologues due to the position of the labeled amino acid

To avoid co-isolation of non-isobaric precursor ions, the minimal mass difference of non-isobaric isotopologues was chosen to be at least 3 Da, translating into 1.5 Th for the predominant doubly charged precursor ion.

**Table 1**

| Peptide name | Sequence | Remarks |
|---|---|---|
| KLK3 peptide_P5 | SLFHP*EDTGQV | T2 loading |
| KLK3 peptide_V11 | SLFHPEDTGQV* | T2 loading |
| KLK3 peptide_F3 | SLF*HPEDTGQV | T2 loading |
| KLK3 peptide_L2V11 | SL*FHPEDTGQV* | T2 loading |
| KLK3 peptide_L2P5 | SL*FHP*EDTGQV | Internal standard |
| KLK3 peptide_L2P5V11 | SL*FHP*EDTGQV* | T2 loading |
| KLK3 peptide_L2F3V11 | SL*F*HPEDTGQV* | T2 loading |

Isotopically labeled peptides were synthesized and further purified using C18-HPLC to a minimum of 85 % purity. Lyophilized peptides were reconstituted in 10% DMSO at a concentration of 1 to 2 mg mL⁻¹. Reconstituted peptide stocks were stored at -80°C until further analysis.

### 2. Acquisition of Isotopologue-Specific Calibration Curves

In order to translate detected relative ion intensities into an absolute measure, isotopologue-specific external calibration curves were acquired in two technical replicates. Therefore, a total of six different isotopologues (Table 1 ; reconstituted as described above) were mixed to a final concentration of 20 pmol µL⁻¹. This stock was subsequently further titrated in 5% formic acid prior to LC/MS analysis. The internal standard KLK3 peptide_L2P5 was added at a constant amount of 5 fmol per LC/MS injection. Samples were separated by reversed-phase chromatography (nanoAcquity UPLC, Waters, Milford, MA) using ACQUITY UPLC BEH C18 columns (75 µm × 250 mm, Waters, Milford, MA) and a gradient ranging from 1 to 34.5% ACN over the course of 35 min. Mass spectrometry was performed on an online coupled Orbitrap Fusion Tribrid mass spectrometer (Thermo Fisher Scientific, Waltham, MA) in scheduled parallel reaction monitoring (sPRM) mode following collisional-induced dissociation (CID) of selected precursor ions. Subsequent peak boundary integration was performed in Skyline (MacLean et al., 2010).

### 3. T2 Cell Culture & Initial Peptide Loading

The T2 cell line was obtained from DSMZ (ACC 598, lot #2). Cells were cultured in RPMI-1640 medium (Gibco, #A1049101) supplemented with 10% heat-inactivated FBS (Gibco, #10270106) in absence of antibiotics, at 37°C in a humidified atmosphere containing 5% CO2. Cells were sub-cultured in 1:4 or 1:6 ratio every 2-3 days. Two days (48 h) before the peptide loading experiment, cell culture medium was changed to RPMI-1640 medium (Gibco, #A1049101) containing 10% heat-inactivated human serum (C.C. Pro, #S-41-M) instead of FBS. Human serum containing RPMI-1640 medium was then used in the peptide loading experiment.

Cells were harvested to ensure overall either 100 or 120 million cells for the experiment. The total amount was distributed among five or six 50-ml Falcon tubes: Each tube finally contained 20 million cells. Following the centrifugation step (1300 rpm, 7 min), each 20-million cell pellet was resuspended in 16 ml of isotopologue dilution (800 µl peptide dilution per one million cells) and transferred to a T75 culture flask for the subsequent 2-h incubation at 37°C in a humidified atmosphere containing 5% CO2. The six T75 flasks were gently shaken every 20-30 min within the incubation period. Following the 2-h incubation with different isotopes, each cell suspension was collected from the corresponding T75 culture flask and transferred to a fresh 50-ml Falcon tube for the subsequent washing steps. Cells were washed with PBS twice (all centrifugation steps were performed at 1300 rpm for 7 min). After the second washing and centrifugation step, the supernatant was removed, and each cell pellet was resuspended in 5 ml of PBS. Subsequently, the cell suspensions from all six tubes were pooled together into one sample (into a fresh 50-ml Falcon tube). Additional volume of 15 ml PBS was used to flush all six tubes to collect any remaining cells. The cell suspension was then centrifuged at 1300 rpm for 7 min, the supernatant was removed, and the final cell pellet was placed directly on dry ice.

### 4. Cytotoxicity Assay & Peptide Loading

The functional avidity of TCRs with calibrated T2 cells was assessed in a co-culture setup. CD8⁺ T cells were pre-stimulated with OKT3 and CD28 and after 3 days electroporated with TCR mRNA. At the day of co-culture Luciferase-transduced T2 cells were loaded with different concentrations of isotopologues of a peptide derived from KLK3. In brief, 2x10⁷ million T2 cells were incubated in 40 ml RPMI + 10% HS supplemented with the respective concentration of peptide for 2h at 37°C, 5% CO2. After the incubation the cells were washed and harvested. A fraction of the cells was used for the co-culture setup and the remaining cells used to determine the absolute copy numbers by AbsQuant^{®} (see method described in WO2016107740A1, the content of which is incorporated herein by reference for enablement purposes). T cells and peptide-loaded T2 cells were seeded at a ratio of 1:1 and incubated for 24h until supernatant harvest. Supernatants were subjected an analysis for the presence of luciferase, released by apoptotic/necrotic T2 cells, killed by peptide-specific T cells. By adding specific substrate, the amount of luciferase present in the supernatant was determined by measuring the chemiluminescent signal in a microplate reader. The functional avidity was assessed by calculating the half maximal killing efficiency of the tested TCRs.

### 5. HLA/peptide affinity purification & LC/MS

Samples were snap-frozen in liquid nitrogen and stored until isolation at -80 °C. After cell lysis in CHAPS detergent buffer, peptide-HLA complexes were isolated by immunoprecipitation using the antibody BB7.2 (Department of Immunology, University of Tübingen, Germany) (Falk et al., 1991) coupled to CNBr-activated Sepharose resin (GE Healthcare Europe, Freiburg, Germany). Peptides were eluted from antibody-resin by acid treatment and purified by ultrafiltration. Prior to LC/MS analysis, the internal standard KLK3 peptide _L2P5 was added at an amount of 5 fmol per injection. LC/MS data acquisition and data analysis were performed as described previously.

### 6. Results

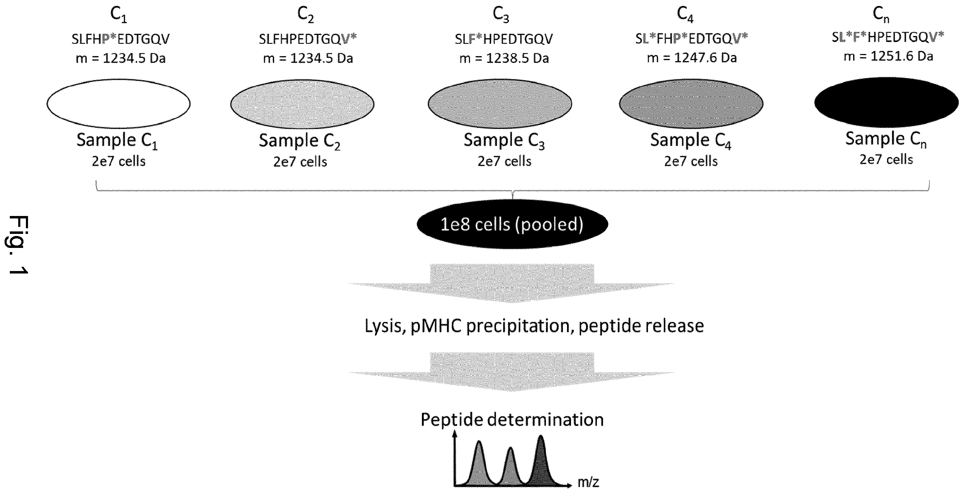

Results are shown in Figs 1 - 6.

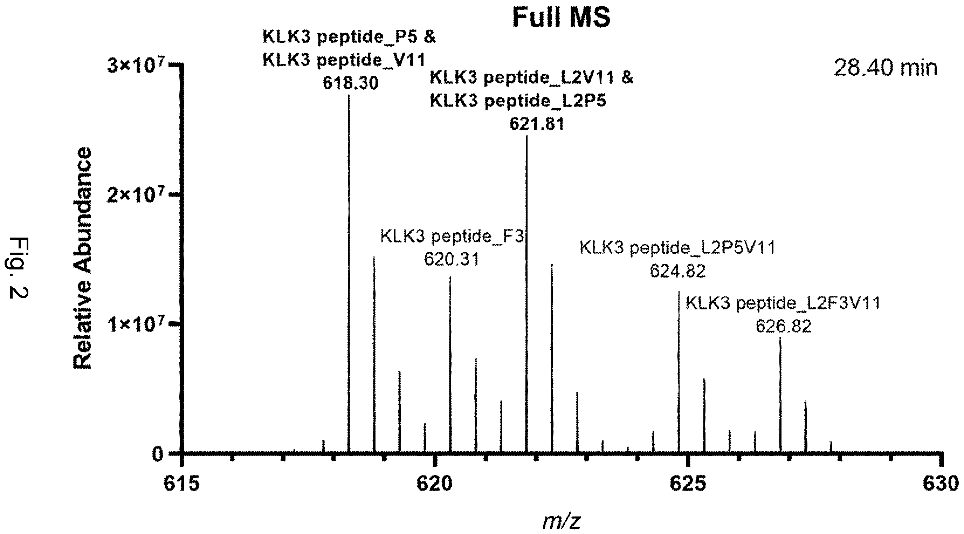

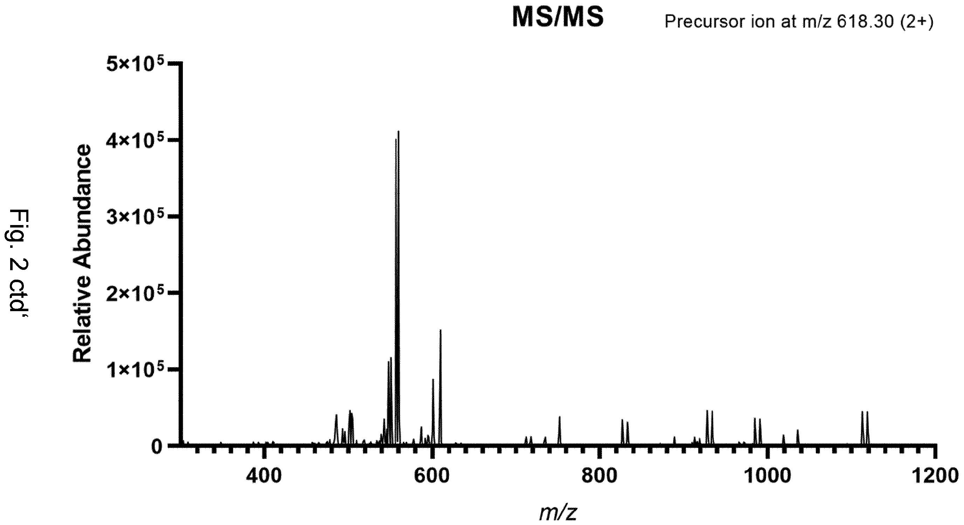

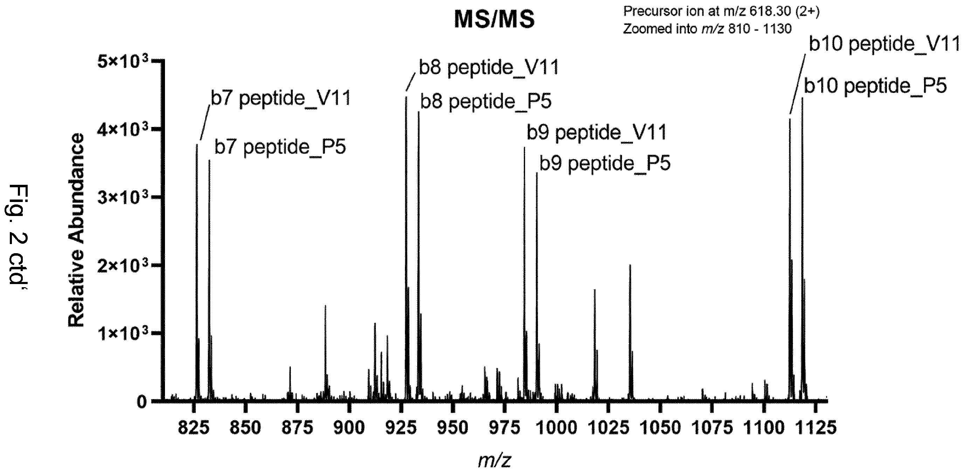

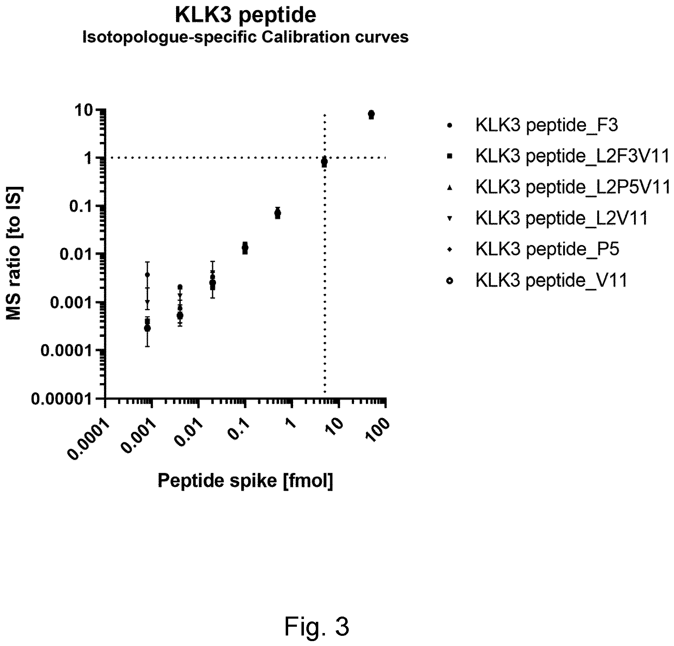

Simultaneous LC/MS analysis of 7 different KLK3 peptide isotopologues at a total injected amount of 5 fmol each revealed the expected pattern, namely that all peptide isoforms were readily distinguishable, either on the MS1 level (Fig 2) or in case that isotopologues were isobaric, on the MS2 level after collisional induced dissociation (CID) by isotopologue-specific fragment ion series, here the formation of a unique b-ion series (Fig 2, lower part). Further titration of respective isotopologues, while KLK3 peptide_L2P5 was kept at 5 fmol, allowed the acquisition of isotopologue-specific calibration curves (Fig 3). These calibration curves allowed for the further conversion of MS signals into an absolute peptide amount (see method described in WO2016107740A1, the content of which is incorporated herein by reference for enablement purposes). Peptide-loading of T2 with 5 different isotopologues cells at a final concentration of 0.1 µg / mL (i.e. ∼ 81 nM) each showed high reproducibility at a CV of 11% ("biological replicates") and translated into an absolute KLK3 peptide abundance of ∼10,000 copies per cell, respectively (Fig 4). In order to further assess the dynamic range, isotopologues were titrated at a range from 0.0001 to 1 µg / mL (i.e. ∼ 0.081 nM to 813 nM) and loaded onto empty T2 cells. Results showed very high linearity at an R² of 0.9988 and revealed that T2 cells were proportionally loaded with KLK3 peptides i.e. a 10-fold higher peptide concentration translated into a 10-fold higher absolute abundance at the given range tested. Further KLK3 peptide L2F3V11 (at a concentration of 0.1 µg mL⁻¹) confirmed previous findings of a peptide abundance ∼ 10,000 copies per cell at this concentration.

Absolute peptide abundance assessment as outlined above of peptide-loaded T2 cells paired with a simultaneously performed cytotoxicity assay further allowed to rank a given set of TCRs tested not only based on their relative functional avidity but also to translate this range into copy number estimates of the respective presented KLK3 peptide (Fig 6).

### Example 2: PRAME Peptide

### Experimental details

Melanoma antigen preferentially expressed in tumors is a protein that in humans is encoded by the PRAME gene. Five alternatively spliced transcript variants encoding the same protein have been observed for this gene.

This gene encodes an antigen that is predominantly expressed in human melanomas and that is recognized by cytolytic T lymphocytes. It is not expressed in normal tissues, except testis. This expression pattern is similar to that of other cancer-testis (CT) antigens, such as MAGE, BAGE, and GAGE. However, unlike these other CT antigens, this gene is also expressed in acute leukemias. The overexpression of PRAME in tumor tissues and relatively low levels in normal somatic tissues make it an attractive target for cancer therapy. In recent years, immunotherapy has spearheaded a new era of cancer therapy resulting in the development of numerous novel antigen-specific immunotherapy approaches. Studies on PRAME-specific immunotherapy primarily involve vaccines and cellular immunotherapies.

PRAME can inhibit retinoic acid signalling and retinoic acid-mediated differentiation and apoptosis. PRAME overexpression in triple negative breast cancer has also been found to promote cancer cell motility through induction of the epithelial-to-mesenchymal transition.

MHC-restricted peptides that are derived from PRAME have for example been disclosed in US10934338B2, the content of which is incorporated herein by reference.

### 1. Isotopologue Selection & Synthesis

A total of 8 isotopologues were selected of which seven were used for T2, Hs695T, and T98G loading experiments and one which served as the internal standard for downstream LC/MS analysis (Table 1). Labeling positions within the respective peptide sequence were chosen based on two criteria of which at least one had to be fulfilled for the unambiguous detection and quantification:
1. Unique precursor mass within the selected set
2. Shared precursor mass but unique fragment ions / transitions compared to other isobaric peptides/isotopologues due to the position of the labeled amino acid

To avoid co-isolation of non-isobaric precursor ions, the minimal mass difference of non-isobaric isotopologues was chosen to be at least 3 Da, translating into 1.5 Th for the predominant doubly charged precursor ion.

**Table 1**

| Peptide name | Sequence | Remarks |
|---|---|---|
| PRAME peptide_L3 | LL*QHLIGL | T2, Hs695T, T98G loading |
| PRAME peptide_L9 | SLLQHLIGL* | T2, T98G loading |
| PRAME peptide_L3L6 | SLL*QHL*IGL | Internal standard |
| PRAME peptide_L3L9 | SLL*QHLIGL* | T2 loading |
| PRAME peptide_L2L3L9 | SL*L*QHLIGL* | T2, Hs695T, T98G loading |
| PRAME peptide_L3L6I7 | SLL*QHL*I*GL | T2, Hs695T, T98G loading |
| PRAME peptide_L2L3L6I7 | SL*L*QHL*I*GL | T2, Hs695T, T98G loading |
| PRAME peptide_L3L6I7L9 | SLL*QHL*I*GL* | T2, Hs695T, T98G loading |

Isotopically labelled peptides were synthesized and further purified using C18-HPLC to a minimum of 85 % purity. Lyophilized peptides were reconstituted in 10% DMSO at a concentration of 1 to 2 mg mL⁻¹. Reconstituted peptide stocks were stored at -80°C until further analysis.

### 2. Acquisition of Isotopologue-Specific Calibration Curves

In order to translate detected relative ion intensities into an absolute measure, isotopologue-specific external calibration curves were acquired in two technical replicates. Therefore, a total of seven different isotopologues (Table 1 ; reconstituted as described above) were mixed to a final concentration of 20 pmol µL⁻¹. This stock was subsequently further titrated in 5% formic acid prior to LC/MS analysis. The internal standard PRAME peptide_L3L6 was added at a constant amount of 100 fmol per LC/MS injection. Samples were separated by reversed-phase chromatography (nanoAcquity UPLC, Waters, Milford, MA) using ACQUITY UPLC BEH C18 columns (75 µm × 250 mm, Waters, Milford, MA) and a gradient ranging from 1 to 34.5% ACN over the course of 35 min. Mass spectrometry was performed on an online coupled Orbitrap Fusion Tribrid mass spectrometer (Thermo Fisher Scientific, Waltham, MA) in scheduled parallel reaction monitoring (sPRM) mode following collisional-induced dissociation (CID) of selected precursor ions. Subsequent peak boundary integration was performed in Skyline (MacLean et al., 2010).

### 3. T2 Cell Culture & Initial Peptide Loading

The T2 cell line was obtained from DSMZ (ACC 598, lot #2). Cells were cultured in RPMI-1640 medium (Gibco, #A1049101) supplemented with 10% heat-inactivated FBS (Gibco, #10270106) in absence of antibiotics, at 37°C in a humidified atmosphere containing 5% CO2. Cells were sub-cultured in 1:4 or 1:6 ratio every 2-3 days. Two days (48 h) before the peptide loading experiment, cell culture medium was changed to RPMI-1640 medium (Gibco, #A1049101) containing 10% heat-inactivated human serum (C.C. Pro, #S-41-M) instead of FBS. Human serum containing RPMI-1640 medium was then used in the peptide loading experiment.

Cells were harvested to ensure overall either 100 or 120 million cells for the experiment. The total amount was distributed among five or six 50-ml Falcon tubes: Each tube finally contained 20 million cells. Following the centrifugation step (1300 rpm, 7 min), each 20-million cell pellet was resuspended in 16 ml of isotopologue dilution (800 µl peptide dilution per one million cells) and transferred to a T75 culture flask for the subsequent 2-h incubation at 37°C in a humidified atmosphere containing 5% CO2. The six T75 flasks were gently shaken every 20-30 min within the incubation period. Following the 2-h incubation with different isotopes, each cell suspension was collected from the corresponding T75 culture flask and transferred to a fresh 50-ml Falcon tube for the subsequent washing steps. Cells were washed with PBS twice (all centrifugation steps were performed at 1300 rpm for 7 min). After the second washing and centrifugation step, the supernatant was removed, and each cell pellet was resuspended in 5 ml of PBS. Subsequently, the cell suspensions from all six tubes were pooled together into one sample (into a fresh 50-ml Falcon tube). Additional volume of 15 ml PBS was used to flush all six tubes to collect any remaining cells. The cell suspension was then centrifuged at 1300 rpm for 7 min, the supernatant was removed, and the final cell pellet was placed directly on dry ice.

### 4. Cytotoxicity Assay of T98G & Peptide Loading of T98G and Hs695T

The functional analysis of PRAME-loaded T98G cells was assessed in a co-culture setup. On the day of co-culture, T98G (or Hs695T) cells were loaded with different concentrations of isotopologues of PRAME peptide. See PCT/EP2020/050936, the content of which is incorporated herein by reference, for enablement purposes regarding details of the co culture. In brief, 2.5x10⁷ T98G (or 2x10⁷ Hs695T) cells were incubated in 40 ml DMEM + 10% FCS supplemented with the respective concentration of peptide for 2h at 37°C, 5% CO2. After the incubation, the cells were washed and harvested. A fraction of the cells (0.5x10⁷) was used for the co-culture setup (T98G) and the remaining cells (all cells for Hs695T) were used to determine the absolute copy numbers by AbsQuant^{®} (see method described in WO2016107740A1, the content of which is incorporated herein by reference for enablement purposes). Human peripheral blood mononuclear cells (PBMCs) and peptide-loaded T98G cells were seeded at a ratio of 10:1 and incubated for 48h in the presence of TCER until supernatant harvest. Supernatants were subjected to an analysis for the presence of lactate dehydrogenase (LDH), released by apoptotic/necrotic T98G cells, killed by peptide-specific T cells. By adding specific substrate, the amount of LDH present in the supernatant was determined by measuring the colorimetric signal in a microplate reader.

### 5. HLA/peptide affinity purification & LC/MS

Samples were snap-frozen in liquid nitrogen and stored until isolation at -80 °C. After cell lysis in CHAPS detergent buffer, peptide-HLA complexes were isolated by immunoprecipitation using the antibody BB7.2 (Department of Immunology, University of Tübingen, Germany) (Falk et al., 1991) coupled to CNBr-activated Sepharose resin (GE Healthcare Europe, Freiburg, Germany). Peptides were eluted from antibody-resin by acid treatment and purified by ultrafiltration. Prior to LC/MS analysis, the internal standard PRAME peptide _L3L6 was added at an amount of 100 fmol per injection. LC/MS data acquisition and data analysis were performed as described previously.

### 6. Results

Results are shown in Figs 7 - 10.

Simultaneous LC/MS analysis of 8 different PRAME peptide isotopologues at a total injected amount of 100 fmol each revealed the expected pattern, namely that all peptide isoforms were readily distinguishable, either on the MS1 level (Fig 7, upper part) or in case that isotopologues were isobaric, on the MS2 level after collisional induced dissociation (CID) by isotopologue-specific fragment ion series, here the formation of a unique b-ion series (Fig 7, lower part). Further titration of respective isotopologues, while PRAME peptide_L3L6 was kept at 100 fmol, allowed the acquisition of isotopologue-specific calibration curves (Fig 8A). These calibration curves allowed for the further conversion of MS signals into an absolute peptide amount (see method described in WO2016107740A1, the content of which is incorporated herein by reference for enablement purposes). In order to further assess the dynamic range, 6 isotopologues were titrated at a range from 0.0001 to 1 µg / mL (i.e. ∼0.1 nM to 1000 nM) and loaded onto empty T2 cells. Results showed very high linearity at an R² of 0.9935 and revealed that T2 cells were proportionally loaded with PRAME peptides i.e. a 10-fold higher peptide concentration translated into a 10-fold higher absolute abundance at the given range tested (Fig 8B). The same applied to peptide-loaded Hs695T (R² of 0.9890, Fig. 8C) and T98G (Fig. 9) cells presenting detectable copy numbers from a loading concentration of 1 nM (∼0.001 µg / mL) PRAME peptide. Absolute peptide abundance assessment as outlined above of peptide-loaded T98G cells paired with a simultaneously performed cytotoxicity assay further allowed to rank a given TCER tested not only based on its relative functional avidity (Fig 10) but also to translate this range into copy number estimates of the respective presented PRAME peptide (Fig 9).

### References

The disclosures of these documents are herein incorporated by reference in their entireties.
Barnstable CJ, Bodmer WF, Brown G, Galfre G, Milstein C, Williams AF, Ziegler A (1978). Production of monoclonal antibodies to group A erythrocytes, HLA and other human cell surface antigens-new tools for genetic analysis. Cell 14, 9-20.
Basu D, Horvath S, O'Mara L, Donermeyer D, Allen PM (2001). Two MHC surface amino acid differences distinguish foreign peptide recognition from autoantigen specificity. J Immunol 166, 4005-4011.
Bercovici N, Duffour MT, Agrawal S, Salcedo M, Abastado JP (2000). New methods for assessing T-cell responses. Clin Diagn Lab Immunol 7, 859-864.
Berger AE, Davis JE, Cresswell P (1982). Monoclonal antibody to HLA-A3. Hybridoma 1, 87-90.
Bhattacharya A, Dorf ME, Springer TA (1981). A shared alloantigenic determinant on Ia antigens encoded by the I-A and I-E subregions: evidence for I region gene duplication. J Immunol 127, 2488-2495.
Boniface JJ, Davis MM (1995). T-cell recognition of antigen. A process controlled by transient intermolecular interactions. Ann N Y Acad Sci 766, 62-69.
Caillat-Zucman S (2009). Molecular mechanisms of HLA association with autoimmune diseases. Tissue Antigens 73, 1-8.
Caron E, Kowalewski DJ, Chiek Koh C, Sturm T, Schuster H, Aebersold R (2015). Analysis of Major Histocompatibility Complex (MHC) Immunopeptidomes Using Mass Spectrometry. Molecular & Cellular Proteomics 14, 3105-3117.
Chames P, Hufton SE, Coulie PG, Uchanska-Ziegler B, Hoogenboom HR (2000). Direct selection of a human antibody fragment directed against the tumor T-cell epitope HLA-A1-MAGE-A1 from a nonimmunized phage-Fab library. Proc Natl Acad Sci U S A 97, 7969-7974.
Chang AY, Gejman RS, Brea EJ, Oh CY, Mathias MD, Pankov D, Casey E, Dao T, Scheinberg DA (2016). Opportunities and challenges for TCR mimic antibodies in cancer therapy. Expert Opin Biol Ther 16, 979-987.
Chowell D, Morris LGT, Grigg CM, Weber JK, Samstein RM, Makarov V, Kuo F, Kendall SM, Requena D, Riaz N, et al. (2018). Patient HLA class I genotype influences cancer response to checkpoint blockade immunotherapy. Science 359, 582-587.
Coley WB (1991). The treatment of malignant tumors by repeated inoculations of erysipelas. With a report of ten original cases. 1893. Clin Orthop Relat Res, 3-11.
Coulie PG, Van Den Eynde BJ, van der Bruggen P, Boon T (2014). Tumour antigens recognized by T lymphocytes: at the core of cancer immunotherapy. Nat Rev Cancer 14, 135-146.
Dahan R, Reiter Y (2012). T-cell-receptor-like antibodies - generation, function and applications. Expert Rev Mol Med 14, e6.
Dao T, Pankov D, Scott A, Korontsvit T, Zakhaleva V, Xu Y, Xiang J, Yan S, de Morais Guerreiro MD, Veomett N, et al. (2015). Therapeutic bispecific T-cell engager antibody targeting the intracellular oncoprotein WT1. Nat Biotechnol 33, 1079-1086.
Davenport AJ, Cross RS, Watson KA, Liao Y, Shi W, Prince HM, Beavis PA, Trapani JA, Kershaw MH, Ritchie DS, et al. (2018). Chimeric antigen receptor T cells form nonclassical and potent immune synapses driving rapid cytotoxicity. Proc Natl Acad Sci U S A 115, E2068-E2076.
Delves PJ, Roitt IM (2000). The immune system. Second of two parts. N Engl J Med 343, 108-117.
DeMars R, Chang CC, Shaw S, Reitnauer PJ, Sondel PM (1984). Homozygous deletions that simultaneously eliminate expressions of class I and class II antigens of EBV-transformed B-lymphoblastoid cells. I. Reduced proliferative responses of autologous and allogeneic T cells to mutant cells that have decreased expression of class II antigens. Hum Immunol 11, 77-97.
Denkberg G, Lev A, Eisenbach L, Benhar I, Reiter Y (2003). Selective targeting of melanoma and APCs using a recombinant antibody with TCR-like specificity directed toward a melanoma differentiation antigen. J Immunol 171, 2197-2207.
Ellis SA, Taylor C, McMichael A (1982). Recognition of HLA-B27 and related antigen by a monoclonal antibody. Hum Immunol 5, 49-59.
Erlich H, Lee JS, Petersen JW, Bugawan T, DeMars R (1986). Molecular analysis of HLA class I and class II antigen loss mutants reveals a homozygous deletion of the DR, DQ, and part of the DP region: implications for class II gene order. Hum Immunol 16, 205-219.
Falk K, Rotzschke O, Stevanovic S, Jung G, Rammensee HG (1991). Allele-specific motifs revealed by sequencing of self-peptides eluted from MHC molecules. Nature 351, 290-296.
Felix NJ, Allen PM (2007). Specificity of T-cell alloreactivity. Nat Rev Immunol 7, 942-953.
Freudenmann LK, Marcu A, Stevanovic S (2018). Mapping the tumour human leukocyte antigen (HLA) ligandome by mass spectrometry. Immunology 154, 331-345.
Gao GF, Willcox BE, Wyer JR, Boulter JM, O'Callaghan CA, Maenaka K, Stuart DI, Jones EY, Van Der Merwe PA, Bell JI, et al. (2000). Classical and nonclassical class I major histocompatibility complex molecules exhibit subtle conformational differences that affect binding to CD8alphaalpha. J Biol Chem 275, 15232-15238.
Goldberg AC, Rizzo LV (2015a). MHC structure and function - antigen presentation. Part 1. Einstein (Sao Paulo) 13, 153-156.
Goldberg AC, Rizzo LV (2015b). MHC structure and function - antigen presentation. Part 2. Einstein (Sao Paulo) 13, 157-162.
Gorga JC, Knudsen PJ, Foran JA, Strominger JL, Burakoff SJ (1986). Immunochemically purified DR antigens in liposomes stimulate xenogeneic cytolytic T cells in secondary in vitro cultures. Cell Immunol 103, 160-173.
Gruen JR, Weissman SM (1997). Evolving views of the major histocompatibility complex. Blood 90, 4252-4265.
Guo HC, Jardetzky TS, Garrett TP, Lane WS, Strominger JL, Wiley DC (1992). Different length peptides bind to HLA-Aw68 similarly at their ends but bulge out in the middle. Nature 360, 364-366.
Hammerling GJ, Rüsch E, Tada N, Kimura S, Hämmerling U (1982). Localization of allodeterminants on H-2Kb antigens determined with monoclonal antibodies and H-2 mutant mice. Proc National Acad Sci 79, 4737-4741.
He Q, Liu Z, Liu Z, Lai Y, Zhou X, Weng J (2019). TCR-like antibodies in cancer immunotherapy. J Hematol Oncol 12, 99.
Hilf N, Kuttruff-Coqui S, Frenzel K, Bukur V, Stevanovic S, Gouttefangeas C, Platten M, Tabatabai G, Dutoit V, van der Burg SH, et al. (2019). Actively personalized vaccination trial for newly diagnosed glioblastoma. Nature 565, 240-245.
Hill AV, Allsopp CE, Kwiatkowski D, Anstey NM, Twumasi P, Rowe PA, Bennett S, Brewster D, McMichael AJ, Greenwood BM (1991). Common west African HLA antigens are associated with protection from severe malaria. Nature 352, 595-600.
Jones B, Janeway CA, Jr. (1981). Cooperative interaction of B lymphocytes with antigen-specific helper T lymphocytes is MHC restricted. Nature 292, 547-549.
Kasuga K (2013). Comprehensive analysis of MHC ligands in clinical material by immunoaffinity-mass spectrometry. Methods Mol Biol 1023, 203-218.
Kirner A, Mayer-Mokler A, Reinhardt C (2014). IMA901: a multi-peptide cancer vaccine for treatment of renal cell cancer. Hum Vaccin Immunother 10, 3179-3189.
Köhler G, Fischer-Lindahl K, Heusser C (1981). Characterization of a monoclonal anti-H-2Kb antibody. The Immune System 2, 202-208.
Kolstad A, Hansen T, Hannestad K (1987). A human-human hybridoma antibody (TrB12) defining subgroups of HLA-DQw1 and -DQw3. Hum Immunol 20, 219-231.
Kowalewski DJ, Stevanovic S (2013). Biochemical large-scale identification of MHC class I ligands. Methods Mol Biol 960, 145-157.
Kuhn NF, Purdon TJ, van Leeuwen DG, Lopez AV, Curran KJ, Daniyan AF, Brentjens RJ (2019). CD40 Ligand-Modified Chimeric Antigen Receptor T Cells Enhance Antitumor Function by Eliciting an Endogenous Antitumor Response. Cancer Cell 35, 473-488 e476.
Lampson LA, Levy R (1980). Two populations of la-like molecules on a human B cell line. J Immunol 125, 293-299.
Lemke H, Hammerling GJ, Hammerling U (1979). Fine specificity analysis with monoclonal antibodies of antigens controlled by the major histocompatibility complex and by the Qa/TL region in mice. Immunol Rev 47, 175-206.
Lewis JW, Neisig A, Neefjes J, Elliott T (1996). Point mutations in the α2 domain of HLA-A2.1 define a functionally relevant interaction with TAP. Current Biology 6, 873-883.
Liu Q, Tian Y, Li Y, Zhang W, Cai W, Liu Y, Ren Y, Liang Z, Zhou P, Zhang Y, et al. (2020). In vivo therapeutic effects of affinity-improved-TCR engineered T-cells on HBV-related hepatocellular carcinoma. Journal for ImmunoTherapy of Cancer 8, e001748.
Ljunggren HG, Karre K (1985). Host resistance directed selectively against H-2-deficient lymphoma variants. Analysis of the mechanism. J Exp Med 162, 1745-1759.
Ljunggren HG, Öhlén C, Höglund P, Franksson L, Kärre K (1991). The RMA-S lymphoma mutant; consequences of a peptide loading defect on immunological recognition and graft rejection. International Journal of Cancer 47, 38-44.
Lustgarten J, Waks T, Eshhar Z (1991). CD4 and CD8 accessory molecules function through interactions with major histocompatibility complex molecules which are not directly associated with the T cell receptor-antigen complex. Eur J Immunol 21, 2507-2515.
MacLean B, Tomazela DM, Shulman N, Chambers M, Finney GL, Frewen B, Kern R, Tabb DL, Liebler DC, MacCoss MJ (2010). Skyline: an open source document editor for creating and analyzing targeted proteomics experiments. Bioinformatics 26, 966-968.
Madden DR, Garboczi DN, Wiley DC (1993). The antigenic identity of peptide-MHC complexes: a comparison of the conformations of five viral peptides presented by HLA-A2. Cell 75, 693-708.
Maeda H, Hirata R (1984). Separation of four class II molecules from DR2 and DRw6 homozygous B lymphoid cell lines. Immunogenetics 20, 639-647.
Molenkamp BG, Vuylsteke RJ, van Leeuwen PA, Meijer S, Vos W, Wijnands PG, Scheper RJ, de Gruijl TD (2005). Matched skin and sentinel lymph node samples of melanoma patients reveal exclusive migration of mature dendritic cells. Am J Pathol 167, 1301-1307.
Neethling FA, Ramakrishna V, Keler T, Buchli R, Woodburn T, Weidanz JA (2008). Assessing vaccine potency using TCRmimic antibodies. Vaccine 26, 3092-3102.
Ozato K, Sachs DH (1981). Monoclonal antibodies to mouse MHC antigens. III. Hybridoma antibodies reacting to antigens of the H-2b haplotype reveal genetic control of isotype expression. J Immunol 126, 317-321.
Parham P, Brodsky FM (1981). Partial purification and some properties of BB7.2. A cytotoxic monoclonal antibody with specificity for HLA-A2 and a variant of HLA-A28. Hum Immunol 3, 277-299.
Rammensee HG (1995). Chemistry of peptides associated with MHC class I and class II molecules. Curr Opin Immunol 7, 85-96.
Rammensee HG, Friede T, Stevanoviic S (1995). MHC ligands and peptide motifs: first listing. Immunogenetics 41, 178-228.
Ray K, Marteyn B, Sansonetti PJ, Tang CM (2009). Life on the inside: the intracellular lifestyle of cytosolic bacteria. Nat Rev Microbiol 7, 333-340.
Rebai N, Mercier P, Kristensen T, Devaux C, Malissen B, Mawas C, Pierres M (1983). Murine H-2Dd-reactive monoclonal antibodies recognize shared antigenic determinant(s) on human HLA-B7 or HLA-B27 molecules or both. Immunogenetics 17, 357-370.
Riberdy JM, Cresswell P (1992). The antigen-processing mutant T2 suggests a role for MHC-linked genes in class II antigen presentation. J Immunol Baltim Md 1950 148, 2586-2590.
Robbins PA, Evans EL, Ding AH, Warner NL, Brodsky FM (1987). Monoclonal antibodies that distinguish between class II antigens (HLA-DP, DQ, and DR) in 14 haplotypes. Hum Immunol 18, 301-313.
Robinson J, Halliwell JA, Hayhurst JD, Flicek P, Parham P, Marsh SG (2015). The IPD and IMGT/HLA database: allele variant databases. Nucleic Acids Res 43, D423-431.
Rock KL, Shen L (2005). Cross-presentation: underlying mechanisms and role in immune surveillance. Immunol Rev 207, 166-183.
Rosenberg SA, Yang JC, Sherry RM, Kammula US, Hughes MS, Phan GQ, Citrin DE, Restifo NP, Robbins PF, Wunderlich JR, et al. (2011). Durable complete responses in heavily pretreated patients with metastatic melanoma using T-cell transfer immunotherapy. Clin Cancer Res 17, 4550-4557.
Royston I, Omary MB, Trowbridge IS (1981). Monoclonal antibodies to a human T-cell antigen and la-like antigen in the characterization of lymphoid leukemia. Transplant P 13, 761-766.
Salter RD, Cresswell P (1986). Impaired assembly and transport of HLA-A and -B antigens in a mutant TxB cell hybrid. EMBO J 5, 943-949.
Salter RD, Howell DN, Cresswell P (1985). Genes regulating HLA class I antigen expression in T-B lymphoblast hybrids. Immunogenetics 21, 235-246.
Schumacher FR, Delamarre L, Jhunjhunwala S, Modrusan Z, Phung QT, Elias JE, Lill JR (2017). Building proteomic tool boxes to monitor MHC class I and class II peptides. Proteomics 17.
Schwanhausser B, Busse D, Li N, Dittmar G, Schuchhardt J, Wolf J, Chen W, Selbach M (2011). Global quantification of mammalian gene expression control. Nature 473, 337-342.
Schwanhausser B, Busse D, Li N, Dittmar G, Schuchhardt J, Wolf J, Chen W, Selbach M (2013). Corrigendum: Global quantification of mammalian gene expression control. Nature 495, 126-127.
Sharma P, Kranz DM (2016). Recent advances in T-cell engineering for use in immunotherapy. F1000Res 5.
Sherman LA, Randolph CP (1981). Monoclonal anti-H-2Kb antibodies detect serological differences between H-2Kb mutants. Immunogenetics 12, 183-186.
Sidney J, Southwood S, Moore C, Oseroff C, Pinilla C, Grey HM, Sette A (2013). Measurement of MHC/peptide interactions by gel filtration or monoclonal antibody capture. Curr Protoc Immunol Chapter 18, Unit 18.13.
Siller-Farfan JA, Dushek O (2018). Molecular mechanisms of T cell sensitivity to antigen. Immunol Rev 285, 194-205.
Spits H, Keizer G, Borst J, Terhorst C, Hekman A, de Vries JE (1983). Characterization of monoclonal antibodies against cell surface molecules associated with cytotoxic activity of natural and activated killer cells and cloned CTL lines. Hybridoma 2, 423-437.
Stern LJ, Wiley DC (1994). Antigenic peptide binding by class I and class II histocompatibility proteins. Structure 2, 245-251.
Storkus WJ, Howell DN, Salter RD, Dawson JR, Cresswell P (1987). NK susceptibility varies inversely with target cell class I HLA antigen expression. J Immunol 138, 1657-1659.
Storkus WJ, Zeh HJ, 3rd, Salter RD, Lotze MT (1993). Identification of T-cell epitopes: rapid isolation of class I-presented peptides from viable cells by mild acid elution. J Immunother Emphasis Tumor Immunol 14, 94-103.
The International HIV Controllers Study, Pereyra F, Jia X, McLaren PJ, Telenti A, de Bakker PI, Walker BD, Ripke S, Brumme CJ, Pulit SL, et al. (2010). The major genetic determinants of HIV-1 control affect HLA class I peptide presentation. Science 330, 1551-1557.
Townsend A, Öhlén C, Bastin J, Ljunggren H-G, Foster L, Kärre K (1989). Association of class I major histocompatibility heavy and light chains induced by viral peptides. Nature 340, 443-448.
Trachtenberg E, Korber B, Sollars C, Kepler TB, Hraber PT, Hayes E, Funkhouser R, Fugate M, Theiler J, Hsu YS, et al. (2003). Advantage of rare HLA supertype in HIV disease progression. Nat Med 9, 928-935.
Urban JL, Schreiber H (1992). Tumor antigens. Annu Rev Immunol 10, 617-644.
van der Merwe PA, Dushek O (2011). Mechanisms for T cell receptor triggering. Nat Rev Immunol 11, 47-55.
Velcheti V, Schalper K (2016). Basic Overview of Current Immunotherapy Approaches in Cancer. Am Soc Clin Oncol Educ Book 35, 298-308.
Whitelegg AM, Oosten LE, Jordan S, Kester M, van Halteren AG, Madrigal JA, Goulmy E, Barber LD (2005). Investigation of peptide involvement in T cell allorecognition using recombinant HLA class I multimers. J Immunol 175, 1706-1714.
Willemsen RA, Ronteltap C, Chames P, Debets R, Bolhuis RL (2005). T cell retargeting with MHC class I-restricted antibodies: the CD28 costimulatory domain enhances antigen-specific cytotoxicity and cytokine production. J Immunol 174, 7853-7858.
Yewdell JW (2003). Immunology. Hide and seek in the peptidome. Science 301, 1334-1335.
Yewdell JW, Anton LC, Bennink JR (1996). Defective ribosomal products (DRiPs): a major source of antigenic peptides for MHC class I molecules? J Immunol 157, 1823-1826.
Zemmour J (1996). Inefficient assembly limits transport and cell surface expression of HLA-Cw4 molecules in C1R. Tissue Antigens 48, 651-661.
Zemmour J, Little AM, Schendel DJ, Parham P (1992). The HLA-A,B "negative" mutant cell line C1R expresses a novel HLA-B35 allele, which also has a point mutation in the translation initiation codon. J Immunol 148, 1941-1948.

### Sequences

The following sequences form part of the disclosure of the present application. A WIPO ST 25 compatible electronic sequence listing is provided with this application, too. For the avoidance of doubt, if discrepancies exist between the sequences in the following table and the electronic sequence listing, the sequences in this table shall be deemed to be the correct ones.

| **SEQ ID No** | **Sequence** | **Qualifier** |
|---|---|---|
| 1 | SLFHPEDTGQV | KLK3 derived peptide |
| 2 | SLLQHLIGL | PRAME derived peptide |

## Claims

1. A method of characterizing the binding characteristics between a peptide of interest and MHC molecules of a given cell type, the method comprising the steps of:
a) Providing two or more cells **characterized by** displaying, on their surface, MHC molecules,
b) dispensing the two or more cells in two or more vessels, so that each vessel comprises one or more cells
c) adding (="loading"), to the different vessels, different variants of a peptide of interest, wherein the variants of said peptide are labeled and have the same amino acid sequence, yet differ from one another in
(iii) the type of labeling, and
(iv) their concentration
and exposing the cells thereto so as to form, in the different vessels, peptide-MHC complexes on the surface of the cells
d) isolating the thus formed peptide-MHC complexes and
e) determining the concentration of the different peptide-MHC complexes formed in step c.

2. The method according to claim 1, wherein the MHC molecule is MHC class I

3. The method according to claim 1 or 2, wherein the peptide of interest has a length of between 8 and 15 amino acid residues.

4. The method according to any one of the aforementioned claims, wherein the variants of the peptide of interest are isotopically labeled ("isotopologues").

5. The method according to any one of the aforementioned claims, wherein the peptide-MHC complexes are isolated by immunoaffinity enrichment, optionally carried out using an MHC-specific antibody.

6. The method according to any one of the aforementioned claims, wherein, after isolation of the peptide-MHC complexes, the peptides are eluted from the MHC, wherein further optionally the concentration of the different peptide variants is determined in the eluate, so as to determine the concentration of the different peptide-MHC complexes formed in step c).

7. The method according to any one of the aforementioned claims, wherein a ratio between
(i) the concentrations of the peptide of interest to which the cells are exposed in step b) and
(ii) the concentrations of different peptide-MHC complexes formed in step c) is determined.

8. The method according to any one of the aforementioned claims, wherein further, for each peptide variant, the cell count of the cells exposed thereto is determined.

9. The method according to any one of the aforementioned claims, wherein the concentration of the different peptide variants is determined on the one or more by means of at least one method selected from the group consisting of
• mass spectrometry (MS)
• tandem mass spectrometry (MS/MS)
• liquid chromatography coupled with mass spectrometry (LC-MS, LC-MS/MS

10. The method according to any one of the aforementioned claims, wherein the peptide that forms part of the peptide-MHC complex is a peptide that is not presented by an established cell line.

11. The method according to any one of the aforementioned claims, wherein the two or more cells **characterized by** displaying, on their surface, MHC molecules, are deficient in peptide antigen processing and/or peptide antigen presentation.

12. The method according to any one of claims 19 - 21, wherein the cell is selected from the group consisting of
• T2 (174xCEM.T2)
• RMA-S
• B-LCL 721.174 or B-LCL 721.180
• C1R-T134K

13. The method according to any one of the aforementioned claims, which method further comprises subjecting at least a part of the cells that have been exposed to the peptide of interest to an assay in which the interaction of a pMHC-binding protein or a pMHC-binding cell to the thus formed peptide-MHC complexes is characterized.

14. The method according to any one of the aforementioned claims, which comprises the determination of a dosage-response relationship related to the interaction between the pMHC-binding protein or the pMHC-binding cell and the pMHC.

15. The method according to any one of claims 13 - 14, wherein the
a) pMHC-binding protein is selected from the group consisting of:
• a T-cell receptor, or a target binding fragment thereof, or
• a TCR-mimic antibody, or a target binding fragment thereof, or
b) the pMHC binding cell is a T cell.
